(19) Europäisches Patentamt
European Patent Office
Office européen des brevets

(11) EP 0 980 236 B1

(12) EUROPÄISCHE PATENTSCHRIFT

(45) Veröffentlichungstag und Bekanntmachung des Hinweises auf die Patenterteilung:
**23.04.2003 Patentblatt 2003/17**

(21) Anmeldenummer: **99937866.4**

(22) Anmeldetag: **26.02.1999**

(51) Int Cl.[7]: **A61K 7/13**

(86) Internationale Anmeldenummer:
**PCT/EP99/01235**

(87) Internationale Veröffentlichungsnummer:
**WO 99/044569 (10.09.1999 Gazette 1999/36)**

(54) **FÄRBEMITTEL UND VERFAHREN ZUR ERZEUGUNG VON TEMPORÄREN HAARFÄRBUNGEN**
COLOURANT AND METHOD FOR PRODUCING TEMPORARY HAIR COLOURS
PRODUIT COLORANT ET PROCEDE DE COLORATION CAPILLAIRE TEMPORAIRE

(84) Benannte Vertragsstaaten:
**CH DE ES FR GB GR IT LI**

(30) Priorität: **06.03.1998 DE 19809646**

(43) Veröffentlichungstag der Anmeldung:
**23.02.2000 Patentblatt 2000/08**

(73) Patentinhaber: **Wella Aktiengesellschaft
64274 Darmstadt (DE)**

(72) Erfinder:
- **GÖTTEL, Otto
CH-1723 Marly (CH)**
- **PIRRELLO, Aline
CH-1762 Givisiez (CH)**

(56) Entgegenhaltungen:
**DE-B2- 2 012 050** **US-A- 5 474 578**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft nicht-oxidative Färbemittel für Haare auf der Basis von bestimmten anionischen Polymethinfarbstoffen sowie ein Verfahren zum temporären Färben von Haaren, bei dem die Färbung zu einem beliebigen Zeitpunkt wieder entfernt werden kann.

Die Färbung von Haaren ist in der heutigen Zeit den unterschiedlichsten Trends unterworfen. Während früher Haare vor allem gefärbt wurden, um Grauanteile zu überdecken, besteht heute immer mehr das Verlangen nach der Integration der Haarfarbe in die aktuelle Mode sowie als Ausdruck der Persönlichkeit.

Zur Färbung von Haaren stehen nach wie vor zwei etablierte Methoden zur Verfügung. Zum einen ist dies die oxidative Haarfärbung, die a priori für temporäre Färbungen ungeeignet ist, da sie zu einem sehr dauerhaften Färbeergebnis führt. Zum anderen besteht die Möglichkeit die Haare mit nicht-oxidativen, direktziehende Farbstoffe enthaltenden Färbemitteln (häufig als Tönungen bezeichnet) zu färben. Obwohl die dafür verwendeten Farbstoffe neben der färberischen Leistung darauf optimiert sind, möglichst lange im Haar zu verbleiben, werden mit jedem Waschvorgang die Tönungsergebnisse graduell schwächer, so daß je nach verwendetem Produkt und Haartyp solche Färbungen in der Regel maximal 10 Haarwäschen überdauern. Falls jedoch ein Benutzer dieser nicht-oxidativen Färbemittel zu einem früheren Zeitpunkt seine ursprüngliche Haarfarbe zurückerhalten möchte, gibt es bisher keine geeigneten Mittel, die die schnelle Wiederherstellung des ursprünglichen Farbtons in zufriedenstellender Weise erlauben, da die dazu eingesetzten Produkte in der Regel sehr aggressiv sind und Haarschädigungen zur Folge haben.

In der Literatur wird eine Vielzahl von Versuchen beschrieben, eine Färbung von Fasern wieder rückgängig zu machen. So wird in der DE-PS 38 42 774 und der US-PS 4 681 471 die Entfärbung von Triarylmethanfarbstoffen mit Reduktionsmitteln beschrieben. In die gleiche Richtung geht auch die US-PS 5 474 578, bei der zur Entfärbung der verwendeten Farbstoffe entweder eine oxidative oder eine reduktive Entfärbung oder aber eine Kombination von beiden Behandlungen verwendet wird. Ein generelles Problem bei diesen Methoden liegt insbesondere darin, daß die Entfärbung meist nur teilweise erfolgt. So ermöglicht das Verfahren nach der US-PS 5 474 578 im günstigsten Fall einen Entfärbegrad von maximal 90 bis 93%, wobei sich dieser Entfärbegrad nur bei einer aufeinanderfolgenden oxidativen und reduktiven Behandlung erzielen läßt. Diese doppelte Behandlung ist jedoch außerordentlich haarschädigend. Normalerweise wird bei diesem Verfahren lediglich eine Teilentfärbung (häufig < 50%) der Haare erzielt.

Den zuletzt genannten Patentanmeldungen ist weiterhin gemeinsam, daß sie sich mit der Entfärbung von in der Haarkosmetik alteingeführten Farbstoffklassen befassen. Die verwendeten Färbemittel basieren auf Direktziehern mit unterschiedlichen chemischen und physikalisch Eigenschaften, welche auch ein unterschiedliches Färbeverhalten und Bleichverhalten besitzen, wodurch spätestens bei auf Farbstoffgemischen aufbauenden Nuancierungen, eine gleichmäßige Entfärbung äußerst problematisch wird, da das Ergebnis von den Eigenschaften der leistungsschwächsten Komponente bestimmt wird.

Es bestand daher ein Bedürfnis nach nicht-oxidativen Färbemitteln, welche jederzeit ohne große Schädigung der Haare wieder entfernt werden können.

Polymethinfarbstoffe so wie deren Eignung zur Färbung von Fasern sind zum Teil bereits seit langem bekannt. Einen Überblick über diese Farbstoffe findet man beispielsweise in Houben-Weyl 5/1d, 4. Auflage (1954), Seite 227 ff. Einige dieser Polymethinfarbstoffe sind zudem im Handel erhältlich. Obwohl zum Teil gute bis befriedigende Farbergebnisse erzielt werden, ist es in der Regel nur schwer möglich, diese Farbstoffe wieder vollständig zu entfernen. So ergeben Pentamethin-Isoxazolonfarbstoffe zwar eine Färbung, eine Entfernung der Färbung ist jedoch nur in einem unzureichenden Maße möglich.

Überraschenderweise wurde nunmehr gefunden, daß Färbemittel auf der Basis von bestimmten anionischen Polymethinfarbstoffen (im folgenden "Oxonolfarbstoffe" genannt), insbesondere Pyrazolon- und Pyridonverbindungen der Formeln (II) bis (IV), eine hervorragende Färbung der Haare ermöglichen, wobei sich die erhaltenen Färbungen auf einfache und schonende Weise innerhalb kurzer Zeit wieder entfernen lassen. Zur Entfärbung sind hierbei sowohl reduzierende als auch oxidierende Mittel geeignet, wobei die Verwendung von reduzierenden Entfärbemitteln bevorzugt ist.

Mit den erfindungsgemäßen Färbemitteln lassen sich Nuancen im abgewandelten Naturton, vor allem aber im modischen Bereich erzielen.

Darüber hinaus ist es möglich, neben den erwähnten Tönen eine Reihe von brillanten Farbreflexen vor allem im hellroten bis blauen Bereich zu erzielen. Bedingt durch die sehr hohe Farbstärke der Farbstoffe und das gute Aufziehverhalten kann in hervorragender Weise der ursprüngliche Farbton der Faser überdeckt werden. Damit kann auch dem eingangs erwähnten Wunsch nach der Integration der Haarfarbe in die Mode sowie als Ausdruck der Persönlichkeit voll entsprochen werden.

Gegenstand der vorliegenden Anmeldung ist daher ein Mittel zur Färbung von Haaren, welches dadurch gekennzeichnet ist, daß es mindestens einen Oxonolfarbstoff der tautomeren Formel (Ia)/(Ib), oder deren physiologisch verträgliche Salze enthält,

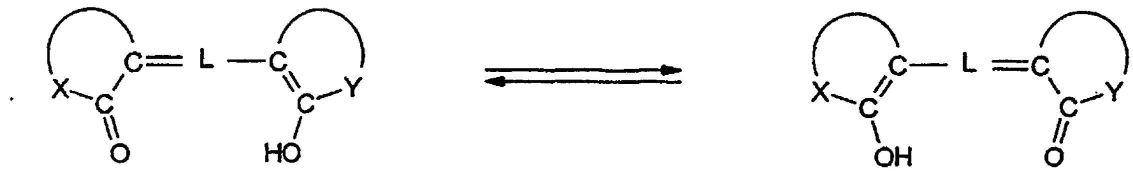

(Ia) (Ib)

wobei in der allgemeinen Formel (Ia)/(Ib) **X** und **Y** unabhängig voneinander jeweils gemeinsam mit den beiden Kohlenstoffatomen des in der Formel (Ia)/(Ib) angegebenen Ringsystems die für die Bildung eines fünfgliedrigen oder sechsgliedrigen heterocyclischen Ringes notwendigen Elemente darstellen und L für eine Brückengruppe der allgemeinen Formel

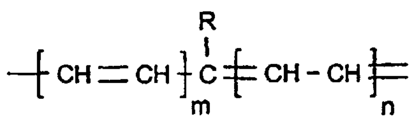

steht, worin **R** ein Wasserstoffatom, eine Phenylgruppe, eine Methylgruppe, eine Carbonamidgruppe oder ein Halogenatom darstellt und die Indices **m** und **n** jeweils gleich 0, 1 oder 2 sein können, wobei die Summe aus m und n nicht größer als 2 ist.

In der allgemeinen Formel (Ia)/(Ib) kann als fünfgliedriger oder sechsgliedriger heterocyclischer Ring eine Pyrazolongruppe, Pyridongruppe, Dioxothiazolingruppe, Rhodaningruppe, Dioxoimidazolidingruppe oder Barbitursäuregruppe verwendet werden; wobei die in der allgemeinen Formel (Ia)/(Ib) enthaltenen beiden Ringsysteme sowohl gleich als auch verschieden sein können. Vorzugsweise sind diese beiden Ringsysteme unter Berücksichtigung der jeweiligen tautomeren Formen identisch und **L** steht für eine Monomethineinheit, Trimethineinheit oder Pentamethineinheit.

Als physiologisch verträgliche Salze der Verbindungen der Formel (Ia)/(Ib) sind insbesondere Alkalisalze und Ammoniumsalze, beispielsweise die Ammoniumsalze, Natriumsalze, Kaliumsalze, N-Methylmorpholiniumsalze, Monoethanolammoniumsalze, Diethanol-ammoniumsalze und Triethanolammoniumsalze, zu nennen, wobei die Natriumsalze, Kaliumsalze und insbesondere die Ammoniumsalze bevorzugt sind.

In ihrer Säureform geschrieben besitzen die Farbstoffe der vorliegenden Erfindung vorzugsweise die Strukturen der allgemeinen Formeln (II) bis (IV), die jeweils in einer ihrer möglichen tautomeren Formen dargestellt sind,

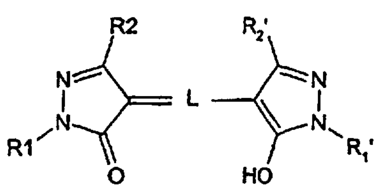

(II),

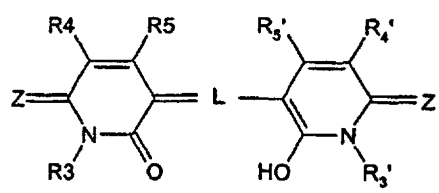

(III),

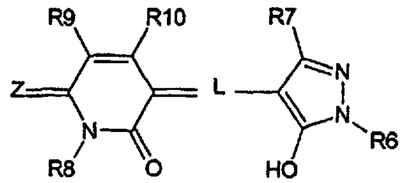

(IV),

worin **R1, R1' und R6** Wasserstoff, eine geradkettige oder verzweigte C1- bis C8- Alkylgruppe, eine Hydroxyethylgruppe, eine Dihydroxypropylgruppe, eine Methoxyethylgruppe, eine Carboxyethylgruppe, eine C1- bis C4-Sulfoalkylgruppe, einen Phenylrest oder einen mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierten Phenylrest, einen Benzylrest oder einen mit einem oder mehreren Halogenatomen, mit einer C1- bis C4-Alkylgruppe, einer Hydroxygruppe, einer Methoxygruppe, einer Carboxygruppe, einer Nitrogruppe oder einer Aminogruppe substituierten Benzylrest oder einen fünfgliedrigen oder sechsgliedrigen gesättigten oder ungesättigten Heterocyclus bedeuten, wobei **R1 und R1'** gleich oder verschieden sein können, und **R2, R2' und R7** Wasserstoff, eine verzweigte oder unverzweigte C1- bis C6- Alkylgruppe, einen Phenylrest, eine Aminogruppe, die zusätzlich acyliert oder sulfonyliert sein kann, eine Acetylgruppe, eine Methoxygruppe, eine Carbonsäuregruppe, die mit einem geradkettigen oder verzweigten C1- bis C8-Alkohol oder einem Ethylenglykolmonomethylether oder einem Ethylenglykolmonoethylether verestert sein kann, eine Carbonsäureamidgruppe, eine Carbonsäureanilidgruppe, eine 2-Amino-2-oxyethylgruppe oder eine Nitrilgruppe bedeuten, wobei **R2 und R2'** gleich oder verschieden sein können, und

**R3, R3' und R8** Wasserstoff, eine geradkettige oder verzweigte C1- bis C11-Alkylgruppe, eine geradkettige oder verzweigte C1- bis C11-Monohydroxyalkylgruppe, eine geradkettige oder verzweigte C1- bis C11-Dihydroxyalkylgruppe, eine geradkettige oder verzweigte C1- bis C11-Alkoxyalkylgruppe, eine geradkettige oder verzweigte C1- bis C11-Monoalkylaminogruppe, eine Aminogruppe der Formel $(CH_2)_x$-NR11R12 (mit x gleich einer ganzen Zahl von 0 bis 3 und R11 und R12 unabhängig voneinander gleich einer C1- bis C3-Alkylgruppe), eine C2- bis C4-Sulfoalkylgruppe oder Carboxyalkylgruppe, eine Phenylgruppe oder eine mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierte Phenylgruppe, eine Benzylgruppe oder eine mit einem oder mehreren Halogenatomen, mit einer C1- bis C4-Alkylgruppe, einer Hydroxygruppe, einer Methoxygruppe, einer Nitrogruppe oder einer Aminogruppe substituierte Benzylgruppe, eine Phenylethylgruppe, ein fünfgliedriger oder sechsgliedriger aromatischer oder nicht-aromatischer Heterocyclus, der direkt oder über eine Methylengruppe gebunden sein kann, eine Pyrrolidinogruppe, Morpholinogruppe , Piperazinogruppe, Piperidinogruppe oder Pyridino(C2- oder C3-)alkylgruppe oder eine Trialkylammoniumalkylgruppe der Formel $R13\text{-}N(R14)_3^+$ (mit R13 gleich einer C1- bis C6-Alkylengruppe und R14 gleich einer Methyl- oder Ethylgruppe, wobei die Gesamtzahl an Kohlenstoffatomen im Molekül gleich 5 bis 9 ist) bedeuten, wobei **R3 und R3'** gleich oder verschieden sein können, und

**R4, R4' und R9** Wasserstoff, eine Nitrilgruppe, eine Carbonsäureestergruppe, eine Carbonsäureamidgruppe, eine Sulfonsäuregruppe, eine Sulfomethylgruppe, eine Methansulfonylgruppe, einen Pyridiniumrest oder einen Imidazoliumrest bedeuten, wobei **R4 und R4'** gleich oder verschieden sein können, und

**R5, R5' und R10** Wasserstoff, eine C1- bis C4-Alkylgruppe, eine C5- bis C6-Cycloalkylgruppe, eine Phenylgruppe, eine Methoxyphenylgruppe, eine Benzylgruppe, eine Phenylethylgruppe oder eine Carbonsäuregruppe bedeuten, wobei **R5 und R5'** gleich oder verschieden sein können, und

**Z** für Sauerstoff oder einen Rest der Formel $C(CN)_2$, $C(CN)COOQ$ oder $C(COOQ)_2$, worin Q jeweils eine C1- bis C8-Alkylgruppe oder einen Ethylenglykolmono(C3- bis C7-)alkylether darstellt, steht, und

**L** für eine Brückengruppe der allgemeinen Formel

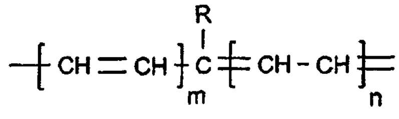

steht, worin **R** ein Wasserstoffatom, eine Phenylgruppe, eine Methylgruppe, eine Carbonamidgruppe oder ein Halogenatom darstellt, und die Indices **m** und **n** jeweils gleich 0, 1 oder 2 sein können, wobei die Summe aus m und n nicht größer als 2 ist.

Besonders bevorzugt sind Farbstoffe der allgemeinen Formeln (II) bis (IV) sowie deren Salze, in denen
**R1 und R1'** gleich sind und Wasserstoff, eine geradkettige oder verzweigte C1- bis C4-Alkylgruppe, eine Hydroxyethylgruppe, eine Dihydroxypropylgruppe, eine Methoxyethylgruppe, eine C2- bis C4-Sulfoalkylgruppe, einen Phenylrest oder einen mit einem oder mehreren Halogenatomen, einer oder zwei Sulfonsäuregruppen, einer Methylgruppe oder einer Methoxygruppe substituierten Phenylrest oder einen Benzylrest oder einen mit einem Halogenatom, mit einer Methylgruppe, Hydroxygruppe, Methoxygruppe, Nitrogruppe oder Aminogruppe substituierten Benzylrest bedeuten und
**R2 und R2'** gleich sind und eine Methylgruppe, eine Aminogruppe, die zusätzlich acyliert sein kann, oder eine Carbonsäuregruppe, die mit einem C1- bis C3-Alkohol verestert sein kann, bedeuten und
**R3 und R3'** gleich oder verschieden sind und Wasserstoff, eine geradkettige C1- bis C4-Alkylgruppe, eine Hydroxyethylgruppe, eine Methoxyethylgruppe, eine Methoxypropylgruppe, eine Sulfoethylgruppe, einen Phenylrest oder einen mit einem Halogenatom, einer Sulfonsäuregruppe, einer Methylgruppe oder Methoxygruppe substituierten Phenylrest, einen Benzylrest oder einen mit einem Halogenatom, mit einer Methylgruppe, Hydroxygruppe, Methoxygruppe, Nitrogruppe oder Aminogruppe substituierten Benzylrest oder einen fünfgliedrigen oder sechsgliedrigen aromatischen oder nicht-aromatischen Heterocyclus, der direkt oder über eine Methylengruppe gebunden sein kann, eine Aminogruppe, eine C1- bis C4-Monoalkyl-aminogruppe, eine Dialkylaminogruppe mit insgesamt 2 bis 8 Kohlenstoffatomen im Molekül oder eine Trialkylammoniumalkylgruppe der Formel $R13\text{-}N(R14)_3^+$ (mit R13 gleich einer C1- bis C6-Alkylengruppe und R14 gleich einer Methyloder Ethylgruppe, wobei die Gesamtzahl an Kohlenstoffatomen im Molekül gleich 5 bis 9 ist) bedeuten und
**R4 und R4'** gleich sind und Wasserstoff, eine Nitrilgruppe, eine Carbonsäureamidgruppe, eine Sulfonsäuregruppe, eine Sulfomethylgruppe, eine Methansulfonylgruppe, eine Pyridiniumgruppe oder eine Imidazoliumgruppe bedeuten und
**R5 und R5'** gleich sind und Wasserstoff, eine Methylgruppe, eine Nitrilgruppe oder einen substituierten oder unsubstituierten Phenylrest bedeuten und
**R6** Wasserstoff, eine geradkettige oder verzweigte C1- bis C4-Alkylgruppe, eine Hydroxyethylgruppe, eine Dihydroxypropylgruppe, eine Methoxyethylgruppe, eine C2- bis C4-Sulfoalkylgruppe, einen Phenylrest oder einen mit einem oder mehreren Halogenatomen, einer oder zwei Sulfonsäuregruppen, einer Methylgruppe oder einer Methoxygruppe substituierten Phenylrest, einen Benzylrest oder einen mit einem Halogenatom, mit einer Methylgruppe, Hydroxygruppe, Methoxygruppe, Nitrogruppe oder Aminogruppe substituierten Benzylrest bedeutet und
**R7** eine Methylgruppe, eine Aminogruppe, die zusätzlich acyliert sein kann, eine Carbonsäuregruppe oder eine mit einem C1- bis C3-Alkohol veresterte Carbonsäuregruppe, bedeutet und
**R8** Wasserstoff, eine geradkettige C1- bis C4-Alkylgruppe, eine Hydroxyethylgruppe, eine Methoxyethylgruppe, eine Sulfoethylgruppe, einen Phenylrest oder einen mit einem Halogenatom, einer Sulfonsäuregruppe, einer Methylgruppe oder Methoxygruppe substituierten Phenylrest, einen Benzylrest oder einen mit einem Halogenatom, mit einer Methylgruppe, Hydroxygruppe, Methoxygruppe, Nitrogruppe oder Aminogruppe substituierten Benzylrest oder einen fünfgliedrigen oder sechsgliedrigen aromatischen oder nicht-aromatischen Heterocyclus, der direkt oder über eine Methylengruppe gebunden sein kann, eine Aminogruppe, eine C1- bis C4-Monoalkylaminogruppe, eine Dialkylaminogruppe mit insgesamt 2 bis 8 Kohlenstoffatomen im Molekül oder eine Trialkylammoniumalkylgruppe der Formel $R13\text{-}N(R14)_3^+$ (mit R13 gleich einer C1- bis C6-Alkylengruppe und R14 gleich einer Methyloder Ethylgruppe, wobei die Gesamtzahl an Kohlenstoffatomen im Molekül gleich 5 bis 9 ist) bedeutet und
**R9** Wasserstoff, eine Nitrilgruppe, eine Carbonsäureamidgruppe, eine Sulfonsäuregruppe, eine Sulfomethylgruppe, eine Pyridiniumgruppe oder eine Imidazoliumgruppe bedeutet und
**R10** Wasserstoff, eine Methylgruppe, eine Nitrilgruppe oder einen substituierten oder unsubstituierten Phenylrest bedeutet und
**Z** gleich Sauerstoff oder einer $C(CN)_2$-Gruppe ist und
**L** eine Brückengruppe der allgemeinen Formel

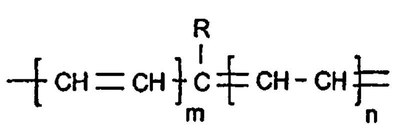

darstellt, worin **R** ein Wasserstoffatom oder eine Methylgruppe darstellt und die indices **m** und **n** jeweils gleich 0, 1 oder 2 sein können, wobei die Summe aus m und n nicht größer als 2 ist.

Als Beispiele für die vorgenannten bevorzugten Farbstoffe der Formeln (II) bis (IV) können die nachfolgend aufgeführten Verbindungen (dargestellt in jeweils einer ihrer möglichen tautomeren Form) genannt werden. In der Säureform geschrieben sind dies:

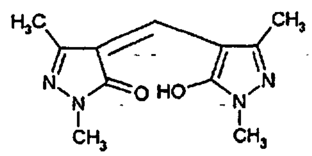

4-(5-Hydroxy-1,3-dimethyl-1H-pyrazol-4-ylmethylen)-2,5-dimethyl-2,4-dihydropyrazol-3-on **(1)**

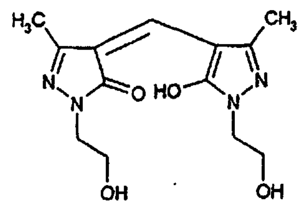

2-(2-Hydroxyethyl)-4-(5-hydroxy-1-(2-hydroxyethyl)-3-methyl-1H-pyrazol-4-ylmethylen)-5-methyl-2,4-dihydropyrazol-3-on **(2)**

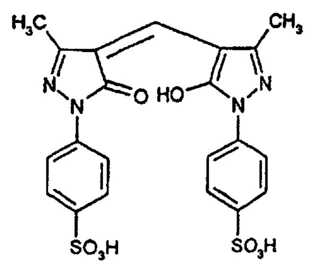

4-(5-Hydroxy-3-methyl-1-(4-sulfophenyl)-1H-pyrazol-4-ylmethylen)-5-methyl-2-(4-sulfophenyl)-2,4-dihydropyrazol-3-on **(3)**

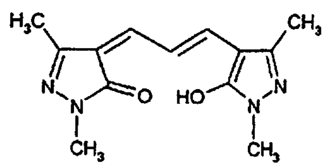

4-(3-(5-Hydroxy-1,3-dimethyl-1H-pyrazol-4-yl)-allyliden)-2,5-dimethyl-2,4-dihydropyrazol-3-on **(4)**

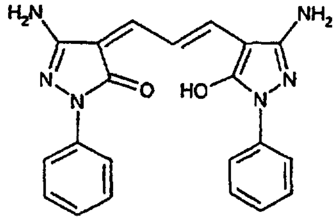

5-Amino-4-(3-(3-amino-5-hydroxy-1-phenyl-1H-pyrazol-4-yl)-allyliden)-2-phenyl-2,4-dihydropyrazol-3-on **(5)**

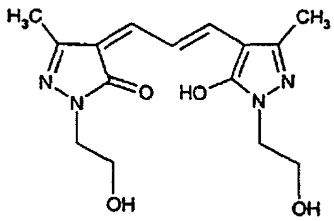

2-(2-Hydroxyethyl)-4-(3-(5-hydroxy-1-(2-hydroxyethyl)-3-methyl-1Hpyrazol-4-yl)-allyliden)-5-methyl-2,4-dihydro-pyrazol-3-on **(6)**

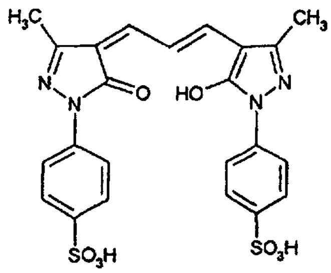

4-(3-(5-Hydroxy-3-methyl-1-(4-sulfophenyl)-1H-pyrazol-4-yl)-allyliden)-5-methyl-2-(4-sulfophenyl)-2,4-dihydro-pyrazol-3-on **(7)**

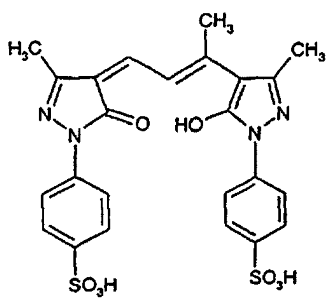

4-(3-(5-Hydroxy-3-methyl-1-(4-sulfophenyl)-1H-pyrazol-4-yl)-but-2-enyliden)-5-methyl-2-(4-sulfophenyl)-2,4-dlhydropyrazol-3-on **(8)**

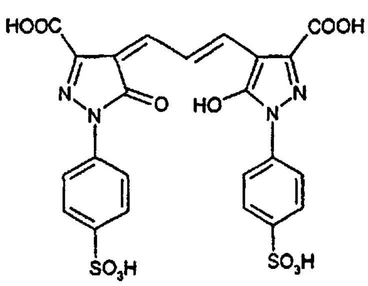

4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure **(9)**

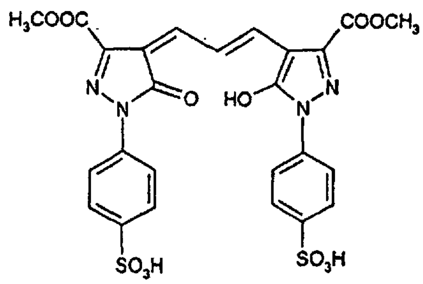

5-Hydroxy-4-(3-(3-methoxycarbonyl-5-oxo-1-(4-sulfophenyl)-1,5-dihydropyrazol-4-yliden)-propenyl)-1-(4-sulfophenyl)-1H-pyrazol-3-carbonsäuremethylester **(10)**

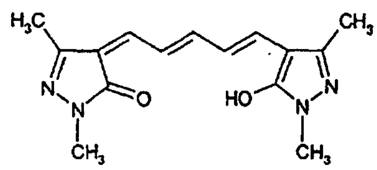

4-(5-(5-Hydroxy-1,3-dimethyl-1H-pyrazol-4-yl)-penta-2,4-dienyliden)-2,5-dimethyl-2,4-dihydropyrazol-3-on **(11)**

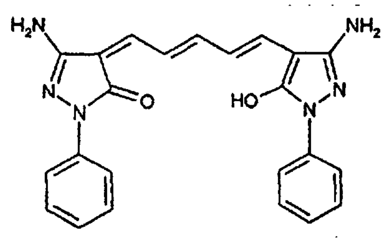

5-Amino-4-(5-(3-amino-5-hydroxy-1-phenyl-1H-pyrazol-4-yl)-penta-2,4-dienyliden)-2-phenyl-2,4-dihydropyrazol-3-on **(12)**

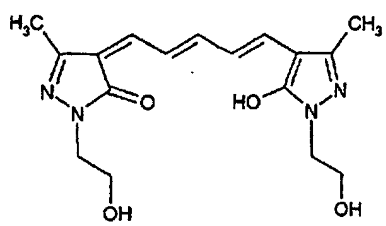

2-(2-Hydroxyethyl)-4-(5-(5-hydroxy-1-(2-hydroxyethyl)-3-methyl-1Hpyrazol-4-yl)-penta-2,4-dienyliden)-5-methyl-2,4-dihydro-pyrazol-3-on **(13)**

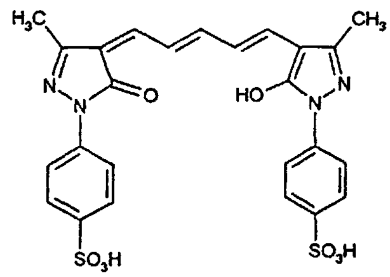

4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on **(14)**

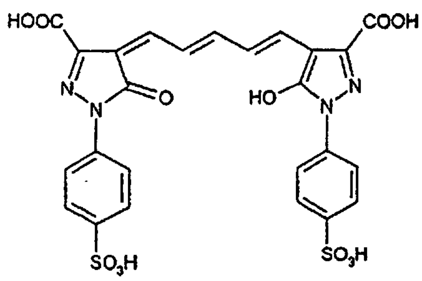

4-(5-(3-Carboxy-5-oxo-1-(4-sulfophenyl)-1,5-dihydropyrazol-4-yliden)-penta-1,3-dienyl)-5-hydroxy-1-(4-sulfophenyl)-1H-pyrazol-3-carbonsäure **(15)**

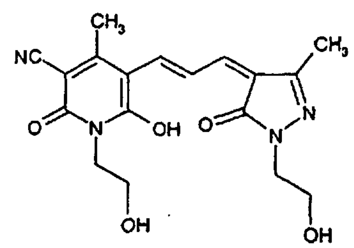

6-Hydroxy-1 -(2-hydroxyethyl)-5-(3-(1-(2-hydroxyethyl)-3-methyl-5-oxo-1,5-dihydro-pyrazol-4-yliden)-propenyl)-4-methyl-2-oxo-1,2-dihydro-pyridin-3-carbonitril **(16)**

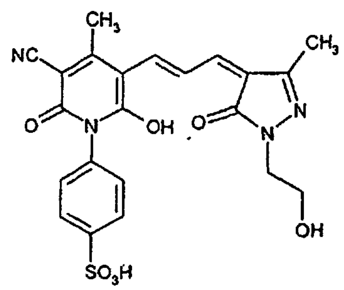

6-Hydroxy-5-(3-(1-(2-hydroxyethyl)-3-methyl-5-oxo-1,5-dihydro-pyrazol-4-yliden)-propenyl)-4-methyl-2-oxo-1-(4-sulfophenyl)-1,2-dihydro-pyridin-3-carbonitril **(17)**

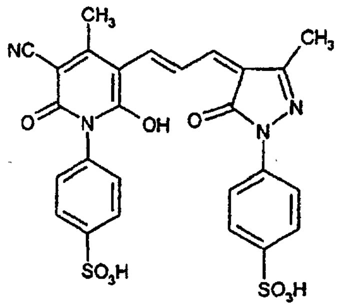

6-Hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril **(18)**

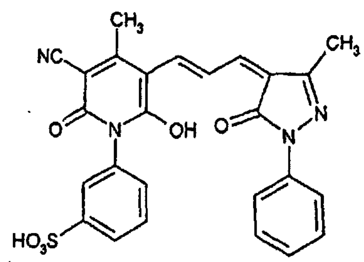

6-Hydroxy-4-methyl-5-(3-(3-methyl-5-oxo-1-phenyl-1,5-dihydropyrazol-4-yliden)-propenyl)-2-oxo-1-(3-sulfophenyl)-1,2-dihydropyridin-3-carbonitril **(19)**

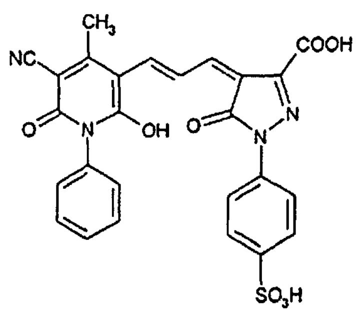

4-(3-(5-Cyano-2-hydroxy-4-methyl-6-oxo-1-phenyl-1,6-dihydropyridin-3-yl)-allyliden)-5-oxo-1-(4-sulfophenyl)-4,5-dihydro-1H-pyrazol-3-carbonsäure **(20)**

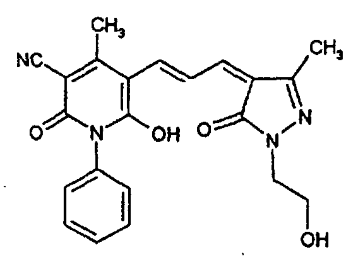

6-Hydroxy-5-(3-(1-(2-hydroxyethyl)-3-methyl-5-oxo-1,5-dihydropyrazol-4-yliden)-propenyl)-4-methyl-2-oxo-1-phenyl-1,2-dihydropyridin-3-carbonitril **(21)**

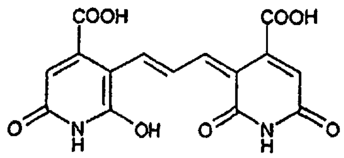

5-(3-(4-Carboxy-2,6-dioxo-1,6-dihydro-2H-pyridin-3-yliden)-propenyl)-6-hydroxy-2-oxo-1,2-dihydropyridin-4-carbonsäure **(22)**

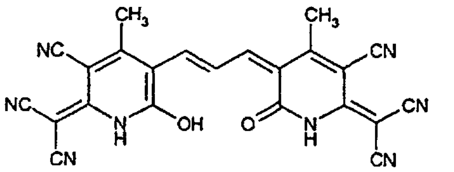

2-(3-Cyano-5-(3-(5-cyano-6-dicyanomethylen-4-methyl-2-oxo-1,6-dihydro-2H-pyridin-3-yliden)-propenyl)-6-hydroxy-4-methyl-1H-pyridin-2-yliden)-malononitril **(23)**

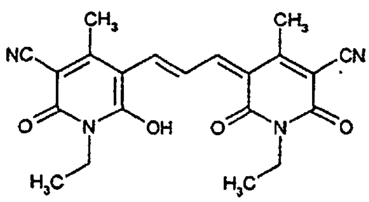

5-(3-(5-Cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(24)**

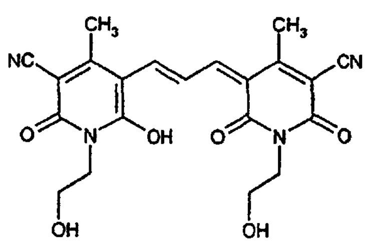

5-(3-(5-Cyano-1-(2-hydroxyethyl)-4-methyl-2,6-dioxo-1,6-dihydro-2Hpyridin-3-yliden)-propenyl)-6-hyd roxy-1-(2-hydroxyethyl)-4-methyl-2-oxo-1,2-dihydropyridin-3-carbonitril **(25)**

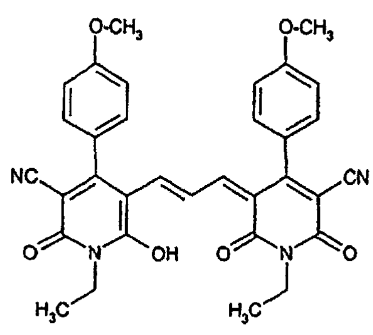

5-(3-(5-Cyano-1-ethyl-4-(4-methoxyphenyl)-2,6-dioxo-1,6-dihydro-2Hpyridin-3-yliden)-propenyl)-1-ethyl-6-hydroxy-4-(4-methoxyphenyl)-2-oxo-1,2-dihydropyridin-3-carbonitril **(26)**

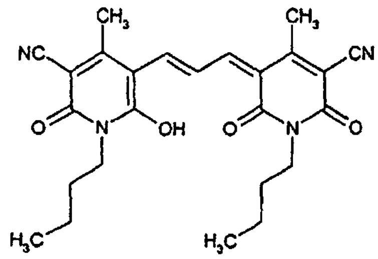

1-Butyl-5-(3-(1-butyl-5-cyano-4-methyl-2,6-dioxo-1,6-dihydro-2H-pyridin-3-yliden)-propenyl)-6-hydroxy-4-methyl-2-oxo-1,2-dihydropyridin-3-carbonitril **(27)**

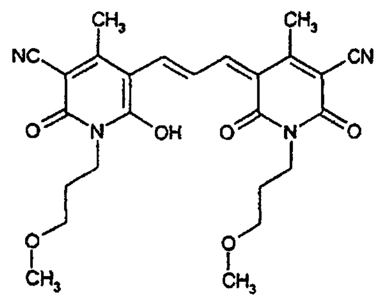

5-(3-(5-Cyano-1-(3-methoxy-propyl)-4-methyl-2,6-dioxo-1,6-dihydro-2Hpyridin-3-yliden)-propenyl)-6-hydroxy-1-(3-methoxy-propyl)-4-methyl-2-oxo-1,2-dihydropyridin-3-carbonitril **(28)**

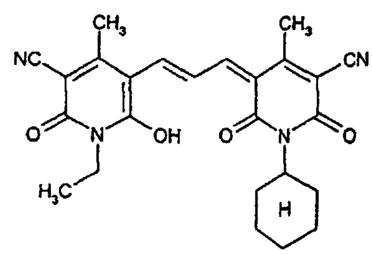

5-(3-(5-Cyano-1-cyclohexyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(29)**

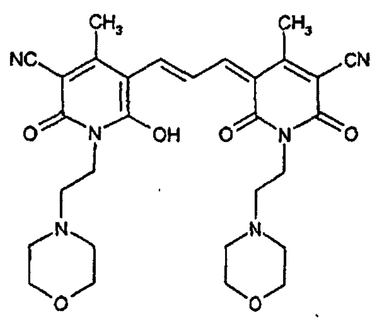

5-(3-(5-Cyano-4-methyl-1-(2-morpholin-4-yl-ethyl)-2,6-dioxo-1,6-dihydro-2H-pyridin-3-yliden)-propenyl)-6-hydroxy-4-methyl-1-(2-morpholin-4-ylethyl)-2-oxo-1,2-dihydropyridin-3-carbonitril **(30)**

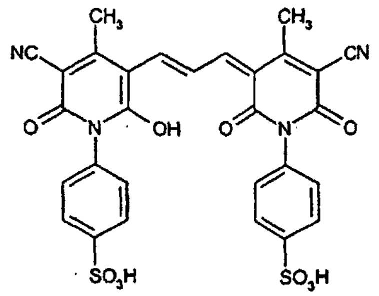

5-(3-(5-Cyano-4-methyl-2,6-dioxo-1-(4-sulfophenyl)-1,6-dihydro-2Hpyridin-3-yliden)-propenyl)-6-hydroxy-4-methyl-2-oxo-1-(4-sulfophenyl)-1,2-dihydropyridin-3-carbonitril **(31)**

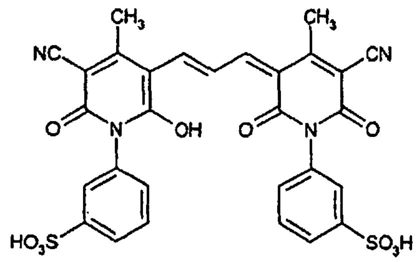

5-(3-(5-Cyano-4-methyl-2,6-dioxo-1-(3-sulfophenyl)-1,6-dihydro-2Hpyridin-3-yliden)-propenyl)-6-hydroxy-4-methyl-2-oxo-1-(3-sulfophenyl)-1,2-dihydropyridin-3-carbonitril **(32)**

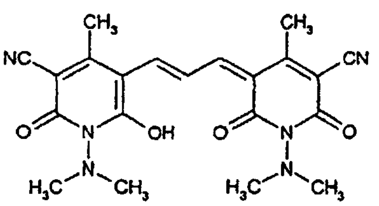

5-(3-(5-Cyano-1-dimethylamino-4-methyl-2,6-dioxo-1,6-dihydro-2Hpyridin-3-yliden)-propenyl)-1-dimethylamino-6-hydroxy-4-methyl-2-oxo-1,2-dihydropyridin-3-carbonitril **(33)**

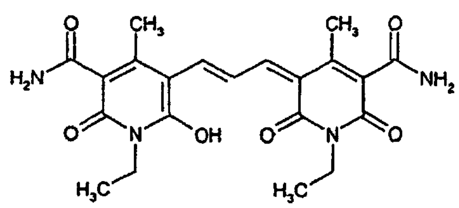

5-(3-(5-Carbamoyl-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonsäureamid **(34)**

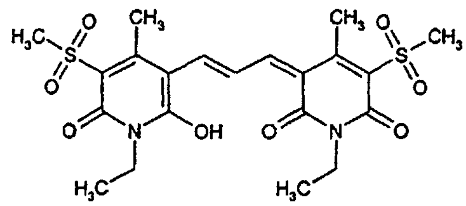

1-Ethyl-3-(3-(1-ethyl-2-hydroxy-5-methansulfonyl-4-methyl-6-oxo-1,6-dihydro-pyridin-3-yl)-allyliden)-5-methansulfonyl-4-methyl-3H-pyridin-2,6-dion **(35)**

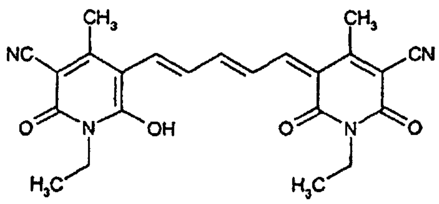

5-(5-(5-Cyano-1-ethyl-4-methyl-2,6-dioxo-1,6-dihydro-2H-pyridin-3-yliden)-penta-1,3-dienyl)-1 -ethyl-6-hydroxy-4-methyl-2-oxo-1,2-dihydropyridin-3-carbonitril **(36)**

Unter den vorgenannten Farbstoffen sind insbesondere Verbindungen der Formel (II) bis (IV) bevorzugt, bei denen

**R1 und R1'** gleich sind und eine Sulfophenylgruppe oder eine Hydroxyethylgruppe bedeuten;
**R2 und R2'** gleich sind und Wasserstoff, eine Methylgruppe, eine Carbonsäuregruppe, eine Carbonsäureestergruppe oder eine Aminogruppe bedeuten;
**R3 und R3'** unabhängig voneinander Wasserstoff oder eine geradkettige C1-C4-Alkylgruppe, eine Methoxyethylgruppe, eine Hydroxyethylgruppe, eine Sulfoethylgruppe, eine Phenylgruppe oder eine Sulfophenylgruppe bedeuten;
**R4 und R4'** gleich sind und Wasserstoff, eine Nitrilgruppe oder eine Carbonsäureamidgruppe bedeuten;
**R5 und R5'** gleich sind und eine Methylgruppe oder eine Carbonsäuregruppe bedeuten;
**R6** eine Phenylgruppe, eine Sulfophenylgruppe, eine Methylgruppe oder eine Hydroxyethylgruppe bedeutet;
**R7** Wasserstoff, eine Methylgruppe, eine Carbonsäuregruppe, eine Carbonsäureestergruppe oder eine Aminogruppe bedeutet;
**R8** Wasserstoff oder eine geradkettige C1-C4-Alkylgruppe, eine Methoxyethylgruppe, eine Hydroxyethylgruppe, eine Sulfoethylgruppe, eine Phenylgruppe oder eine Sulfophenylgruppe bedeutet;
**R9** eine Nitrilgruppe bedeutet;
**R10** eine Methylgruppe oder eine Carbonsäuregruppe bedeutet;
**Z** gleich Sauerstoff ist;
**R** einem Wasserstoffatom oder einer Methylgruppe entspricht und
die Indices **m** und **n** jeweils gleich 0, 1 oder 2 sein können, wobei die Summe aus **m** und **n** maximal den Wert 2 ergibt.

Bei Farbstoffen mit ausgeprägtem anionogenen Charakter (dies gilt vor allem für die Farbstoffe, die Säurefunktionen wie Sulfonsäure- oder Carbonsäuregruppen enthalten) können diese Farbstoffe auch in Form der physiologisch verträglichen Salze eingesetzt werden. Insbesondere können die folgenden Verbindungen genannt werden:

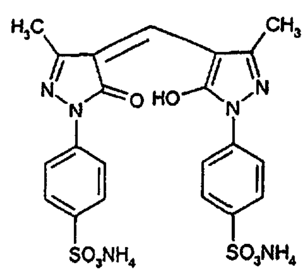

Diammonium-4-(5-hydroxy-3-methyl-1-(4-sulfophenyl)-1H-pyrazol-4-ylmethylen)-5-methyl-2-(4-sulfophenyl)-2,4-dihydro-pyrazol-3-on **(3a)**

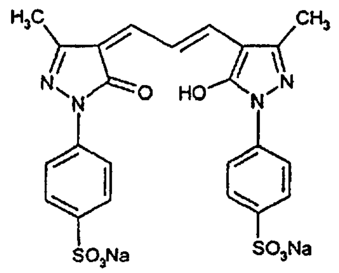

Dinatrium-4-(3-(5-hydroxy-3-methyl-1-(4-sulfophenyl)-1H-pyrazol-4-yl)-allyliden)-5-methyl-2-(4-sulfophenyl)-2,4-dihydro-pyrazol-3-on **(7a)**

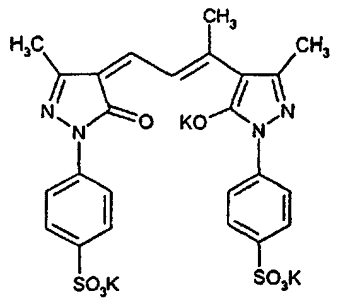

Trikalium-4-(3-(5-hydroxy-3-methyl-1-(4-sulfophenyl)-1H-pyrazol-4-yl)-but-2-enyliden)-5-methyl-2-(4-sulfophenyl)-2,4-dihydro-pyrazol-3-on **(8a)**

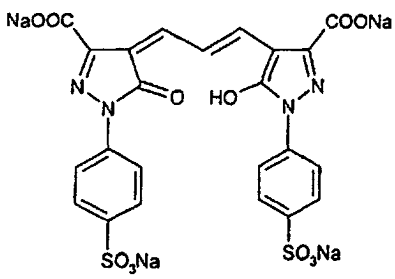

Tetranatrium-4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure **(9a)**

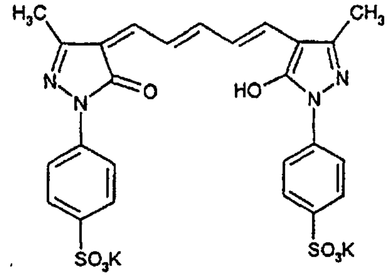

Dikalium-4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on **(14a)**

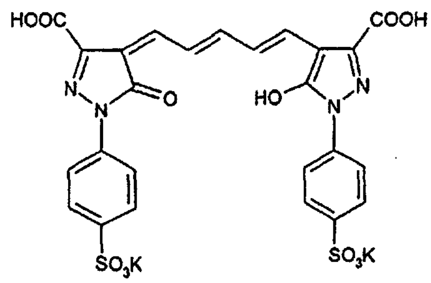

Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure **(15a)**

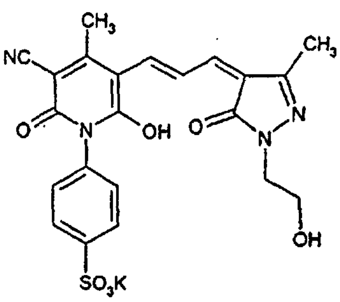

Kalium-6-hydroxy-5-(3-(1-(2-hydroxyethyl)-3-methyl-5-oxo-1,5-dihydropyrazol-4-yliden)-propenyl)-4-methyl-2-oxo-1-(4-sulfophenyl)-1,2-dihydropyridin-3-carbonitril **(17a)**

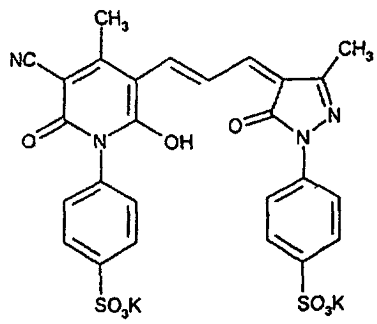

Dikalium-6-hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril **(18a)**

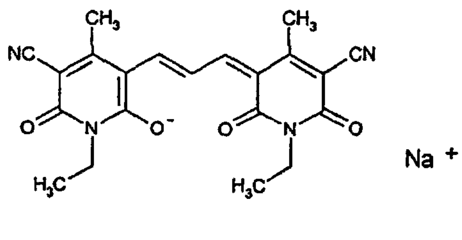

Natrium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril **(24a)**

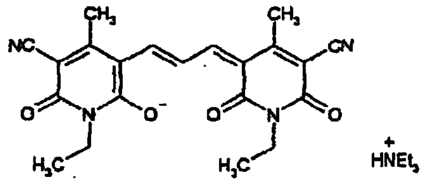

Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril

Der Gesamtgehalt an den Oxonolfarbstoffen der Formel (Ia/Ib) bis (IV), beträgt in dem erfindungsgemäßen Mittel zur Färbung von Keratinfasem 0,01 bis 5 Gewichtsprozent, vorzugsweise 0,5 bis 4 Gewichtsprozent.

Zur Erhöhung der Farbintensität können die in kosmetischen Systemen üblichen Carrier zugesetzt werden. Geeignete Verbindungen sind beispielsweise Benzylalkohol, Vanillin oder Isovanillin. Weitere geeignete Carrier werden in der DE-OS 196 18 595 beschrieben, auf die hiermit ausdrücklich Bezug genommen wird.

Das erfindungsgemäße Haarfärbemittel kann beispielsweise in Form einer Lösung, insbesondere als wäßrigalkoholische Lösung, einer Creme, eines Geles oder einer Emulsion. vorliegen. Als Lösungsmittel können neben Wasser beispielsweise niedere aliphatische einwertige oder mehrwertige Alkohole, deren Ester und Ether, oder aber Gemische dieser Lösungsmittel untereinander oder mit Wasser genannt werden. Der maximale Siedepunkt der vorgenannten geeigneten Lösungsmittel beträgt etwa 400°C, wobei ein Siedepunkt von 20°C bis 250°C bevorzugt ist.

Ebenfalls ist es möglich, dieses Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder als Aerosolschaum aus einem Druckbehälter zu entnehmen.

Der pH-Wert des erfindungsgemäßen Färbemittels beträgt 2 bis 11, wobei ein pH-Wert von 2,5 bis 8 besonders bevorzugt ist. Die Einstellung eines alkalischen pH-Wertes erfolgt vorzugsweise mit Ammoniak, es ist jedoch auch möglich, anstelle von Ammoniak organische Amine, wie zum Beispiel Monoethanolamin oder Triethanolamin zu verwenden. Für die Einstellung eines sauren pH-Wertes kann hingegen eine organische oder anorganische Säure, wie zum Beispiel Salzsäure, Schwefelsäure, Phosphorsäure, Ascorbinsäure, Glycolsäure oder Milchsäure, verwendet werden.

Selbstverständlich kann das vorstehend beschriebene Färbemittel gegebenenfalls weitere für deartige Färbemittel übliche Zusätze, wie zum Beispiel Pflegestoffe, Netzmittel, Verdicker, Weichmacher, Konservierungsstoffe und Parfümöle sowie auch weitere, nachstehend aufgeführte Zusätze enthalten.

Weiterhin können in dem erfindungsgemäßen Färbemittel Netzmittel oder Emulgatoren aus den Klassen der anionischen, amphoteren, nichtionogenen oder zwitterionischen oberflächenaktiven Substanzen wie Fettalkoholsulfate, Alkansulfonate, Alkylbenzolsulfonate, Alkylbetaine, $\alpha$-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkemmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein.

Das vorstehend beschriebene Färbemittel kann weiterhin natürliche oder synthetische Polymere beziehungsweise modifizierte Polymere natürlichen Ursprungs enthalten sein, wodurch gleichzeitig mit der Färbung eine Festigung der Keratinfaser erreicht wird. Solche Mittel werden im allgemeinen als Tönungsfestiger oder Farbfestiger bezeichnet. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (deacetyliertes Chitin) oder Chitosanderivate, eingesetzt werden können.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent. Die vorgenannten Polymere können in dem erfindungsgemäßen Mittel in der für solche Mittel üblichen Menge, insbesondere in einer Menge von etwa 1 bis 5 Gewichtsprozent, verwendet werden.

Für die Haarfärbung wird das erfindungsgemäße Färbemittel in üblicher Weise in einer für die Färbung der Haare ausreichenden Menge, im allgemeinen etwa 50 bis 150 Gramm, auf das Haar aufgetragen. Nach einer für die Haarfärbung ausreichenden Einwirkungszeit, die üblicherweise etwa 10 bis 45 Minuten bei 20 bis 50 °C, vorzugsweise 15 bis 30 Minuten bei etwa 40°C, beträgt, wird das Haar mit Wasser gespült, gegebenenfalls mit einem Shampoo gewaschen und/oder mit der wäßrigen Lösung einer schwachen organischen Säure, wie zum Beispiel Zitronensäure oder Weinsäure, nachgespült und getrocknet.

Die Anwendung des Färbemittels mit zusätzlicher Festigung erfolgt in bekannter und üblicher Weise durch Befeuchten des Haares mit dem Festiger, Einlegen des Haares zur Frisur und anschließende Trocknung.

Hinsichtlich der färberischen Möglichkeiten bietet das erfindungsgemäße Haarfärbemittel je nach Art und Zusammensetzung der verwendeten Oxonolfarbstoffe eine breite Palette verschiedener Farbnuancen, die sich von natürlichen Farbtönen bis hin zu hochmodischen, leuchtenden Nuancen erstreckt. Die vorzüglichen Eigenschaften der neuen Farbstoffe zeigen sich insbesondere auf durch Licht und Wetter geschädigtem oder auf dauergewellten Haaren.

Da in dem erfindungsgemäßen Färbemittel ausschließlich Oxonolfarbstoffe, welche als strukturanaloge Verbindungen alle zueinander kompatibel sind, eingesetzt werden, wird ein gleichmäßiges Färbe- und Entfärbeergebnis erhalten.

Das erfindungsgemäße Färbesystem zeichnet sich weiterhin dadurch aus, daß die Haarfärbung zu jedem beliebigen Zeitpunkt wieder rückgängig gemacht werden kann. Von besonderem Vorteil ist hierbei, daß bei der Entfärbung die ursprüngliche Pigmentierung des behandelten Haares wiederhergestellt wird, wobei es in der Praxis unerheblich ist, ob die ursprüngliche Pigmentierung die natürliche Haarfarbe ist oder aber durch eine oxidative Haarfärbung erzielt wurde. Hierdurch ist es möglich sowohl die natürliche Haarfarbe als auch eine permanente Haaarfärbung für einen selbst gewählten Zeitraum zu ändern und nach Ablauf dieses Zeitraums in praktisch unveränderter Form wieder zurück zu erhalten.

Gegenstand der vorliegenden Anmeldung ist daher auch ein Verfahren zum temporären Färben von Haaren ("ON/OFF-Tönung"), bei dem das ungefärbte oder aber ein oxidativ gefärbtes Haar mit dem erfindungsgemäßen Färbemittel in der vorstehend beschriebenen Weise gefärbt wird und später ( zu einem beliebigen vom Anwender gewünschten Zeitpunkt) mit einem Reduktionsmittel oder Oxidationsmittel wieder entfärbt wird.

Die Oxonolfarbstoffe der Formel (Ia/Ib) bis (IV) können beispielsweise reduktiv durch Einwirkung von geeigneten Reduktionsmitteln, wie zum Beispiel Sulfiten, Pyrosulfiten oder Hydrogensulfiten, beispielsweise Alkalisulfiten, Alkalihydrogensulfiten oder Alkalipyrosulfiten (wie zum Beispiel Natriumsulfit, Natriumpyrosulfit, Kaliumpyrosulfit), Ammoniumsulfit oder Ammoniumhydrogensulfit, oder geeigneten Oxidationsmitteln, wie zum Beispiel handelsüblichen, Persulfate enthaltenden Blondierpulvern, vollständig entfärbt werden. Die Blondierpulver weisen in der Regel einen Gehalt von 5 bis 50 Gewichtsprozent, vorzugsweise 15 bis 30 Gewichtsprozent, Ammoniumpersulfat oder Alkalipersulfate oder einer Mischung aus Ammoniumpersulfat und Alkalipersulfaten auf. Die Entfärbung erfolgt vorzugsweise reduktiv unter Verwendung der vorgenannten Entfärbemittel (insbesondere mit Ammoniumsulfit oder Ammoniumhydrogensulfit), wobei die Verwendung der vorgenannten Reduktionsmittel in Kombination mit anderen Reduktionsmitteln, wie zum Beispiel Reduktonen und/oder Thiolen, besonders bevorzugt ist.

Die Einwirkungszeit des Entfärbemittels beträgt hierbei je nach zu entfärbender Färbung und Temperatur (etwa 20 bis 50 Grad Celsius) 5 bis 45 Minuten, insbesondere 5 bis 30 Minuten, wobei durch Wärmezufuhr der Entfärbeprozeß beschleunigt werden kann. Nach Beendigung der Einwirkungszeit des Entfärbemittels wird das Haar mit Wasser gespült, gegebenenfalls mit einem Shampoo gewaschen und/oder einer Spülung, vorzugsweise einer neutralen oder schwach sauren Spülung, behandelt und sodann getrocknet, wobei sowohl das Shampoo als auch die Spülung ein Redukton, wie zum Beispiel Ascorbinsäure, enthalten kann.

Man erhält so nach der Entfärbung den ursprünglichen Farbton zurück. Verwendet man hingegen das Blondiermittel mit einer Wasserstoffperoxidlösung, vorzugsweise einer 6- bis 12prozentigen Wasserstoffperoxidlösung, so werden zum einen die Oxonolfarbstoffe vollständig entfärbt, zum anderen aber wird das Haar zusätzlich aufgehellt ("blondiert").

In einer bevorzugten Ausführungsform werden das erfindungsgemäße Haarfärbemittel und das Entfärbemittel in Form eines 2-Komponenten-Kits konfektioniert, wobei es besonders vorteilhaft ist, wenn das Entfärbemittel speziell auf das zur Färbung verwendete erfindungsgemäße Haarfärbemittel abgestimmt ist.

Die Herstellung von Monomethinoxonolfarbstoffen (m = n = 0) kann aus den entsprechenden Heterocyclen und Trialkyl-orthoestern oder Formamiden als Brückenvorstufen erfolgen. Zahlreiche Verbindungen sind beschrieben bei B. Schied, J. prakt. Chem. **157,** Seite 203 ff. (1941) und S. Hünig, Annalen, **574,** Seite 106 ff. (1951) sowie der dort zitierten Literatur. Zur Herstellung der Trimethinoxonole (m + n = 1) können nach der DE-OS 20 12 050 Tetramethoxypropan, Trimethoxypropen oder Malonaldehyddianil verwendet werden. Pentamethinoxonole (m + n = 2) lassen sich aus N-akzeptorsubstituierten Pyridiniumsalzen unter Ringöffnung des Pyridinrings ("Zincke-Spaltung") oder einem entsprechenden Derivat, wie zum Beispiel Glutaconaldehyddianil, herstellen. Eine komplette Zusammenfassung darüber findet man in Houben-Weyl 7/1, 4. Auflage (1954) auf den Seiten 263 ff. sowie der dort zitierten Literatur.

Die nachstehenden Beispiele sollen den Erfindungsgegenstand näher erläutern, ohne ihn auf die angeführten

Beispiele zu beschränken.

**Beispiele**

**Beispiel 1:** Verfahren zur Herstellung von Diammonium-4-(5-hydroxy-3-methyl-1-(4-sulföphenyl)-1H-pyrazol-4-ylmethylen)-5-methyl-2-(4-sulfophenyl)-2,4-dihydro-pyrazol-3-on **(3a)**

50,85 g 3-Methyl-1-(4-sulfophenyl)-2-pyrazolin-5-on und 30,8 g Ammoniumacetat werden in 400 ml Essigsäure suspendiert und auf 90°C erwärmt. Unter Rühren tropft man 10,6 g Trimethylorthoformiat zu, wobei sich die Reaktionsmischung spontan gelb färbt. Man rührt noch 4 Stunden weiter, kühlt auf Raumtemperatur ab und saugt den ausgefallenen Farbstoff ab. Nachwaschen mit Isopropanol und trocknen liefert 50,8 g Diammonium-4-(5-hydroxy-3-methyl-1-(4-sulfophenyl)-1H-pyrazol-4-ylmethylen)-5-methyl-2-(4-sulfophenyl)-2,4-dihydro-pyrazol-3-on **(3a)** in Form eines leuchtend gelben Produktes.

| | |
|---|---|
| Schmelzpunkt: | > 250 °C |
| $\lambda_{max}$ ($H_2O$) : | 432 nm |
| ε: | 36500 |
| $^1$H-NMR ($D_2O$): | δ = 7,70 (d, $^3J_{HH}$ = 8,6 Hz, 4H); 7,59 (d, $^3J_{HH}$ = 8,6 Hz, 4H); 6,89 (s, 1H); 4,68 (HOD-Austauschsignal); 1,89 ppm (s, 6H). |

**Beispiel 2:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,7 g | Diammonium-4-(5-Hydroxy-3-methyl-1-(4-sulfophenyl)-1H-pyrazol-4-ylmethylen)-5-methyl-2-(4-sulfophenyl)-2,4-dihydro-pyrazol-3-on **(3a)** |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist leuchtend gelb gefärbt.

**Beispiel 3:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,4 g | Trikalium-4-(3-(5-hydroxy-3-methyl-1-(4-sulfophenyl)-1H-pyrazol-4-yl)-but-2-enyliden)-5-methyl-2-(4-sulfophenyl)-2,4-dihydro-pyrazol-3-on **(8a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Das Haar ist leuchtend rot gefärbt.

**Beispiel 4:** Färbemittel

| | |
|---|---|
| 5,0 g | Ethanol |

(fortgesetzt)

| | |
|---|---|
| 1,5 g | Glycolsäure |
| 2,0 g | Natrium-cocoamphoacetat (50%ige wäßrige Lösung) |
| 5,0 g | Benzylalkohol |
| 1,2 g | Natrium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril **(24a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Das Haar ist intensiv blau gefärbt.

**Beispiel 5:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,8 g | Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure **(15a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Das Haar ist blaugrün gefärbt.

**Beispiel 6:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 0,7 g | Diammonium-4-(5-Hydroxy-3-methyl-1-(4-sulfophenyl)-1H-pyrazol-4-ylmethylen)-5-methyl-2-(4-sulfophenyl)-2,4-dihydro-pyrazol-3-on **(3a)** |
| 0,2 g | Tetranatrium-4-(3-(3-carboxy-5-hydroxy-1-(4-sulfophenyl)-1H-pyrazol-4-yl)-prop-2-enyliden)-5-carboxy-2-(4-sulfophenyl)-2,4-dihydro-pyrazol-3-on **(9a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Das Haar erhält einen mittleren Kastanienton.

**Beispiel 7:** Reduktive Entfärbung von mit Oxonolfarbstoffen gefärbten Fasern

**a.)** Zur Entfärbung wird das gemäß den Beispielen 2 bis 6 gefärbte Haar jeweils mit einer 10 prozentigen wäßrigen Ammoniumbisulfit-Lösung behandelt. Nach wenigen Minuten tritt die Entfärbung ein. Das Haar wird mit Wasser gründlich ausgespült und anschließend getrocknet. Das Haar erhält in allen Fällen seine ursprüngliche Farbe zurück (97- bis 99%ige Entfärbung).

**b.)** Zusätzlich wurden die Entfärbbarkeit von anderen Fasem (Seide, Nylon 66) mit der von Haaren verglichen. Hierzu wurden die Fasern jeweils mit einem Färbemittel gemäß Beispiel 2 bis 6 gefärbt und anschließend in der unter a.) beschriebenen Weise entfärbt, wobei aufgrund der schlechten Entfärbbarkeit der anderen Fasern eine

Entfärbedauer von 30 Minuten gewählt wurde. Das Ergebnis ist in der nachfolgenden Tabelle zusammengefaßt.

Im Gegensatz zu Haar, welches vollständig entfärbt werden kann, ist bei Seide (welche ebenfalls eine Keratinfaser ist) und Nylon 66 (einer synthetischen Faser), wenn überhaupt, nur eine teilweise Entfärbung möglich.

| **gemäß Beispiel 2 gefärbte Fasern** | **L*** | **a*** | **b*** | **$\Delta E_{1/2}$** | **Entfärbungs- grad [%]** |
|---|---|---|---|---|---|
| Haar | 86,23 | -0,91 | 11,77 | | |
| gefärbt | 81,77 | -4,87 | 79,05 | 67,54 | |
| entfärbt | 84,32 | -2,17 | 13,72 | 65,44 | 97 |
| Seide | 85,69 | 1,33 | 9,50 | | |
| gefärbt | 80,16 | 0,34 | 82,83 | 73,94 | |
| entfärbt | 84,30 | -4,74 | 45,26 | 38,14 | 52 |
| Nylon 66 | 91,71 | -0,67 | 5,50 | | |
| gefärbt | 83,09 | 0,62 | 81,82 | 76,82 | |
| entfärbt | 88,72 | -10,47 | 64,32 | 21,47 | 28 |

| **gemäß Beispiel 3 gefärbte Fasern** | **L*** | **a*** | **b*** | **$\Delta E_{1/2}$** | **Entfärbungs- grad [%]** |
|---|---|---|---|---|---|
| Haar | 86,23 | -0,91 | 11,77 | | |
| gefärbt | 19,76 | 14,86 | -25,62 | 77,88 | |
| entfärbt | 84,71 | -1,42 | 10,94 | 76,29 | 98 |
| Seide | 85,69 | 1,33 | 9,50 | | |
| gefärbt | 31,42 | 47,39 | 8,03 | 71,53 | |
| entfärbt | 45,41 | 61,49 | -8,26 | 25,69 | 36 |
| Nylon 66 | 91,71 | -0,67 | 5,50 | | |
| gefärbt | 38,21 | 51,20 | -5,39 | 75,31 | |
| entfärbt | 54,89 | 66,00 | -12,50 | 23,41 | 31 |

| **gemäß Beispiel 4 gefärbte Fasern** | **L*** | **g*** | **b*** | **$\Delta E_{1/2}$** | **Entfärbungs- grad [%]** |
|---|---|---|---|---|---|
| Haar | 86,23 | -0,91 | 11,77 | | |
| gefärbt | 19,76 | 14,86 | -25,62 | 77,88 | |
| entfärbt | 84,71 | -1,42 | 8,94 | 75,35 | 97 |
| Nylon 66 | 91,71 | -0,67 | 5,50 | | |
| gefärbt | 22,25 | 22,83 | -34,77 | 83,66 | |
| entfärbt | 32,12 | 38,37 | -62,73 | 33,48 | 40 |
| Seide | 85,69 | 1,33 | 9,50 | | |
| gefärbt | 24,05 | 15,20 | -20,23 | 69,93 | |
| entfärbt | 65,83 | -0,13 | -21,30 | 44,52 | 64 |

| **gemäß Beispiel 5 gefärbte Fasern** | **L*** | **a*** | **b*** | **$\Delta E_{1/2}$** | **Entfärbungs- grad [%]** |
|---|---|---|---|---|---|
| Haar | 86,23 | -0,91 | 11,77 | | |
| gefärbt | 27,83 | -3,84 | -17,81 | 65,53 | |
| entfärbt | 85,60 | -2,09 | 11,09 | 64,62 | 99 |

(fortgesetzt)

| gemäß Beispiel 5 gefärbte Fasern | L* | a* | b* | $\Delta E_{1/2}$ | Entfärbungs- grad [%] |
|---|---|---|---|---|---|
| Seide | 85,69 | 1,33 | 9,50 | | |
| gefärbt | 24,85 | 0,41 | -9,92 | 63,98 | |
| entfärbt | 77,26 | -8,80 | 9,66 | 56,70 | olivgrün[1)] |
| Nylon 66 | 91,71 | -0,67 | 5,50 | | |
| gefärbt | 34,52 | -6,35 | -15,50 | 61,19 | |
| entfärbt | 56,33 | -20,57 | -13,61 | 26,10 | 43 |

[1)] Keine Entfärbung der Faser, sondern Farbumschlag

| gemäß Beispiel 6 gefärbte Fasern | L* | a* | b* | $\Delta E_{1/2/3}$ | Entfärbungs- grad [%] |
|---|---|---|---|---|---|
| Haar | 86,23 | -0,91 | 11,77 | | |
| gefärbt | 31,46 | 20,46 | -2,99 | 60,62 | |
| entfärbt | 81,51 | -4,14 | 15,79 | 58,85 | 97 |
| Seide | 85,69 | 1,33 | 9,50 | | |
| gefärbt | 36,22 | 45,46 | 7,48 | 66,66 | |
| entfärbt | 84,42 | -2,86 | 34,77 | 73,50 | gelb[1)] |
| Nylon 66 | 91,71 | -0,67 | 5,50 | | |
| gefärbt | 49,84 | 41,20 | 11,37 | 59,50 | |
| entfärbt | 69,63 | 37,24 | 31,72 | 28,66 | orange[1)] |

[1)] Keine Entfärbung der Faser, sondern Farbumschlag

**Beispiel 8:** Oxidative Entfärbung der mit Oxonolfarbstoffen gefärbten Haare

Zur Entfärbung wird ein mit einem Färbemittel gemäß Beispiel 4 gefärbtes mittelblondes Haar mit einem handelsüblichen 25prozentigen wäßrigen Bleichmittel (Ammoniumpersulfat/Alkalipersulfat-Kombination 1:1) ohne Wasserstoffperoxid-Zusatz behandelt. Nach einer Einwirkungszeit von wenigen Minuten wird das Haar mit Wasser gründlich ausgespült und getrocknet. Das Haar besitzt wieder seine ursprüngliche Farbe.

| | L* | a* | b* | $\Delta E_{1/2}$ | Entfärbungs- grad [%] |
|---|---|---|---|---|---|
| Haar | 36,75 | 7,25 | 12,71 | | |
| gefärbt | 18,68 | 3,88 | -6,81 | 26,15 | |
| entfärbt | 36,00 | 8,10 | 13,33 | 26,90 | 100 |

**Beispiel 9:** Oxidative Entfärbung und gleichzeitige Bleichung der mit Oxonolfarbstoffen gefärbten Haare

Mit einem Färbemittel gemäß Beispiel 4 gefärbte mittelblonde Haare werden zur Entfärbung und gleichzeitigen Blondierung mit einer 1:1-Mischung aus einem handelsüblichen 50-prozentigen wäßrigen Bleichmittel (Ammoniumpersulfat/Alkalipersulfat-Kombination 1:1) und einer 6prozentigen wäßrigen Wasserstoffperoxid-Lösung behandelt. Nach einer Einwirkungszeit von 10 Minuten wird das Haar mit Wasser gründlich ausgespült und getrocknet. Das Haar wird auf diese Weise gleichzeitig entfärbt und zusätzlich blondiert.

| | L* | a* | b* | $\Delta E_{1/2}$ | Entfärbungs-grad [%] * |
|---|---|---|---|---|---|
| Haar | 36,75 | 7,25 | 12,71 | | |
| gefärbt | 18,68 | 3,88 | -6,81 | 26,15 | |
| entfärbt | 56,73 | 9,37 | 29,22 | 52,69 | 202 |

*) Anmerkung: Der berechnete Entfärbegrad von 202 % setzt sich zusammen aus der Summe der Entfärbung des Oxonolfarbstoffs sowie der zusätzlichen Aufhellung der natürlichen Pigmentierung ("Blondierung").

**Beispiel 10:** Färbemittel

| | |
|---|---|
| 5,0 g | Ethanol |
| 1,5 g | Glycolsäure |
| 2,0 g | Natrium-cocoamphoacetat (50prozentige wäßrige Lösung) |
| 5,0 g | Benzylalkohol |
| 1,4 g | Dinatrium-4-(3-(5-hydroxy-3-methyl-1-(4-sulfophenyl)-1 H-pyrazol-4-yl)-allyliden)-5-methyl-2-(4-sulfophenyl)-2,4-dihydro-pyrazol-3-on **(7a)** |
| 0,2 g | Dikalium-6-hydroxy-4-methyl-5-(3-(3-methyl-5-oxo-1-(4-sulfophenyl)-1,5-dihydropyrazol-4-yliden)-propenyl)-2-oxo-1 -(4-sulfophenyl)-1,2-dihydropyridin-3-carbonitril**(18a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Man erhält ein in einem helleren Kupferton gefärbtes Haar.

Zur Entfärbung wird das Haar mit einer 10prozentigen wäßrigen Natriumsulfitlösung behandelt. Nach wenigen Minuten tritt die Entfärbung ein. Das Haar wird anschließend gut ausgewaschen und getrocknet. Das Haar behält einen hellbeigen Schimmer.

| | L* | a* | b* | $\Delta E_{1/2}$ | Entfärbungs grad [%] |
|---|---|---|---|---|---|
| Haar | 66,23 | -0,91 | 11,77 | | |
| gefärbt | 40,19 | 39,35 | 21,55 | 61,94 | |
| entfärbt | 79,06 | 8,29 | 1,42 | 53,67 | 87 |

**Beispiel 11:** Färbemittel

| | |
|---|---|
| 5,0 g | Ethanol |
| 1,5 g | Glycolsäure |
| 2,0 g | Natrium-cocoamphoacetat (50prozentige wäßrige Lösung) |
| 5,0 g | Benzylalkohol |
| 1,4 g | Diammonium-4-(5-hydroxy-3-methyl-1-(4-sulfophenyl)-1H-pyrazol-4-ylmethylen)-5-methyl-2-(4-sulfophenyl)-2,4-dihydro-pyrazol-3-on **(3a)** |
| 0,5 g | Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure **(15a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Man erhält ein grün gefärbtes Haar.
Zur Entfärbung wird das Haar mit einer 10prozentigen wäßrigen Natriumbisulfitlösung behandelt. Nach wenigen Minuten tritt die Entfärbung ein. Das Haar wird anschließend gut ausgewaschen und getrocknet. Das Haar behält einen hellbeigen Schimmer.

| | L* | a* | b* | $\Delta E_{1/2}$ | Entfärbungs grad [%] |
|---|---|---|---|---|---|
| Haar | 86,23 | -0,91 | 11,77 | | |
| gefärbt | 44,10 | -30,15 | 14,76 | 51,37 | |
| entfärbt | 83,05 | -9,21 | 17,59 | 44,31 | 86 |

**Beispiel 12:** Färbemittel

| | |
|---|---|
| 5,0 g | Ethanol |
| 1,5 g | Glycolsäure |
| 2,0 g | Natrium-cocoamphoacetat (50prozentige wäßrige Lösung) |
| 5,0 g | Benzylalkohol |
| 1,4 g | Diammonium-4-(5-hydroxy-3-methyl-1-(4-sulfophenyl)-1H-pyrazol-4-ylmethylen)-5-methyl-2-(4-sulfophenyl)-2,4-dihydro-pyrazol-3-on **(3a)** |
| 0,2 g | Tetranatrium-4-(3-(3-carboxy-5-hydroxy-1-(4-sulfophenyl)-1 H-pyrazol-4-yl)-prop-2-enyliden)-5-carboxy-2-(4-sulfophenyl)-2,4-dihydro-pyrazol-3-on **(9a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Man erhält ein in einem mittleren Kastanienton gefärbtes Haar.

Zur Entfärbung wird das Haar mit einer einer wäßrigen Lösung behandelt, die 5% Natriumsulfit und 5% Ascorbinsäure enthält. Nach wenigen Minuten tritt die Entfärbung ein. Das Haar wird anschließend gut ausgewaschen und getrocknet. Das Haar behält einen blaßgelben Schimmer.

| | L* | a* | b* | $\Delta E_{1/2}$ | Entfärbungs grad [%] |
|---|---|---|---|---|---|
| Haar | 86,23 | -0,91 | 11,77 | | |
| gefärbt | 55,46 | 40,80 | 37,16 | 57,72 | |
| entfärbt | 85,18 | -2,42 | 18,08 | 55,81 | 97 |

**Beispiel 13:** Färbemittel

| | |
|---|---|
| 5,0 g | Ethanol |
| 1,5 g | Glycolsäure |
| 2,0 g | Natrium-cocoamphoacetat (50prozentige wäßrige Lösung) |
| 5,0 g | Benzylalkohol |
| 0,8 g | Diammonium-4-(5-hydroxy-3-methyl-1-(4-sulfophenyl)-1H-pyrazol-4-ylmethylen)-5-methyl-2-(4-sulfophenyl)-2,4-dihydro-pyrazol-3-on **(3a)** |
| 0,2 g | Dikalium-6-hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril **(18a)** |
| 0,7 g | Tetranatrium-4-(3-(3-carboxy-5-hydroxy-1-(4-sulfophenyl)-1H-pyrazol-4-yl)-prop-2-enyliden)-5-carboxy-2-(4-sulfophenyl)-2,4-dihydro-pyrazol-3-on **(9a)** |
| 0,4 g | Dikalium-4-(3-(5-hydroxy-3-methyl-1-(4-sulfophenyl)-1H-pyrazol-4-yl)-penta-2-enyliden)-5-methyl-2-(4-sulfophenyl)-2,4-dihydro-pyrazol-3-on **(14a)** |
| ad 100,0 g | Wasser, demineralisiert |

Die Färbelösung wird auf gebleichtes Haar aufgetragen. Nach 20 Minuten Einwirkzeit bei 40°C wird mit Wasser ausgewaschen und getrocknet. Man erhält ein anthrazit gefärbtes Haar.
Zur Entfärbung wird das Haar mit einer 10prozentigen wäßrigen Ammoniumbisulfitlösung behandelt. Nach wenigen

Minuten tritt die Entfärbung ein. Das Haar wird anschließend gut ausgewaschen und getrocknet. Das Haar behält einen blaßgelben Schimmer.

| | L* | a* | b* | $\Delta E_{1/2}$ | Entfärbungs grad % |
|---|---|---|---|---|---|
| Haar | 86,23 | -0,91 | 11,77 | | |
| gefärbt | 25,36 | 10,75 | -5,11 | 64,23 | |
| entfärbt | 81,66 | 3,18 | 9,11 | 58,56 | 91 |

**Beispiel 14:** Umfärbung auf oxidativ gefärbtem Haar

Gebleichtes Haar, das mit einem handelsüblichen Oxidationshaarfärbemittel mittelblond gefärbt worden ist, wird mit einem Färbemittel gemäß Beispiel 4 behandelt. Nach 20 Minuten Einwirkzeit bei 40°C wird mit Wasser ausgewaschen und getrocknet. Das Haar ist in einem intensiven Auberginetons gefärbt.

Zur Entfärbung wird das Haar mit einer 10prozentigen wäßrigen Ammoniumbisulfitlösung behandelt. Nach wenigen Minuten erhält man den Farbton des vorgefärbten Haares bei einer zusätzlichen Aufhellung zurück.

| | L* | a* | b* | $\Delta E_{1/2}$ | Entfärbungs grad [%] |
|---|---|---|---|---|---|
| oxidativ gefärbtes Haar | 37,24 | 4,76 | 12,78 | | |
| zusätzlich gefärbt | 21,84 | -1,41 | -0,45 | 21,22 | |
| entfärbt | 45,00 | 5,01 | 20,33 | 31,77 | 150*) |

*) Anmerkung: Der berechnete Entfärbegrad von 150 % setzt sich zusammen aus der Summe der Entfärbung des Oxonolfarbstoffs sowie der zusätzlichen Aufhellung der zuvor applizierten Oxidationshaarfarbe.

**Beispiel 15:** Umfärbung auf oxidativ gefärbtem Haar

Gebleichtes Haar, das mit einem handelsüblichen Oxidationshaarfärbemittel mittelblond gefärbt worden ist, wird mit dem folgenden Färbemittel behandelt.

| | |
|---|---|
| 5,0 g | Ethanol |
| 1,5 g | Glycolsäure |
| 2,0 g | Natrium-cocoamphoacetat (50 prozentige wäßrige Lösung) |
| 5,0 g | Benzylalkohol |
| 1,4 g | Dinatrium-4-(3-(5-hydroxy-3-methyl-1-(4-sulfophenyl)-1 H-pyrazol-4-yl)-allyliden)-5-methyl-2-(4-sulfophenyl)-2,4-dihydro-pyrazol-3-on **(7a)** |
| ad 100,0 g | Wasser, demineralisiert |

Nach 20 Minuten Einwirkzeit bei 40°C wird mit Wasser ausgewaschen und getrocknet. Das Haar ist in einem ziegelroten Ton gefärbt.

Zur Entfärbung wird das Haar mit einer 10prozentigen wäßrigen . Ammoniumbisulfitlösung behandelt. Nach wenigen Minuten erhält man den Farbton des vorgefärbten Haares bei einer zusätzlichen Aufhellung zurück.

| | L* | a* | b* | $\Delta E_{1/2}$ | Entfärbungs grad [%] |
|---|---|---|---|---|---|
| oxidativ gefärbtes Haar | 37,24 | 4,76 | 12,78 | | |
| zusätzlich | | | | 26,78 | |
| gefärbt | 26,58 | 29,16 | 9,96 | | |
| entfärbt | 47,53 | 4,51 | 22,00 | 34,52 | 129*) |

*) Anmerkung: Der berechnete Entfärbegrad von 129 % setzt sich zusammen aus der Summe der Entfärbung des Oxonolfarbstoffs sowie der zusätzlichen Aufhellung der oxidativen Farbe ("Blondierung").

**Beispiel 16:** Färbemittel mit Carrier

| | |
|---|---|
| 5,0 g | Ethanol |
| 2,0 g | Milchsäure |
| 2,0 g | Natrium-cocoamphoacetat (50prozentige wäßrige Lösung) |
| 5,0 g | Carrier gemäß nachfolgender Tabelle |
| 1,2 g | Natrium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril **(24a)** |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Mittel ohne Carrier ergibt einem mittleren Blauton, die Mittel mit Carrier führen zu intensiven Blautönen.

| Beispiel | Carrier | L* | a* | b* |
|---|---|---|---|---|
| **a** | ohne | 35,18 | 26,57 | -46,26 |
| **b** | Vanillin | 19,20 | 19,13 | -28,43 |
| **c** | 2-Hydroxymethyl-thiophen | 18,22 | 18,77 | -26,96 |
| **d** | Benzylalkohol | 18,88 | 13,13 | -22,19 |

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert $\Delta E$ gibt die Farbdifferenz an, die zwischen dem unbehandelten und dem gefärbten Haar beziehungsweise dem gefärbten und dem entfärbten Haar besteht. Er wird folgendermaßen bestimmt:

$$\Delta E_1 = \sqrt{(L_1 - L_0)^2 + (a_1 - a_0)^2 + (b_1 - b_o)^2}$$

$$\Delta E_2 = \sqrt{(L_2 - L_1)^2 + (a_2 - a_1)^2 + (b_2 - b_1)^2}$$

wobei $L_0$, $a_0$ und $b_0$ die Farbmesswerte vor der Färbung beziehungsweise Entfärbung bedeuten, $L_1$, $a_1$ und $b_1$ die Werte nach der Färbung und $L_2$, $a_2$ und $b_2$ die Werte nach der Entfärbung bedeuten.

Der Entfärbegrad in Prozent wurde nach der folgenden Formel ermittelt:

$$\text{Entfärbegrad [\%]} = (\Delta E_2 / \Delta E_1) \times 100$$

Hierbei steht $\Delta E_1$ für den Färbeschritt und $\Delta E_2$ für den Entfärbeschritt.

Alle in der vorliegenden Anmeldung genannten Prozentangaben stellen soweit nicht anders angegeben Gewichtsprozente dar.

## Patentansprüche

1. Mittel zur Färbung von Keratinfasem, **dadurch gekennzeichnet, daß** es mindestens einen Oxonolfarbstoff der tautomeren Formel (Ia)/(Ib) oder deren physiologisch verträgliche Salze enthält,

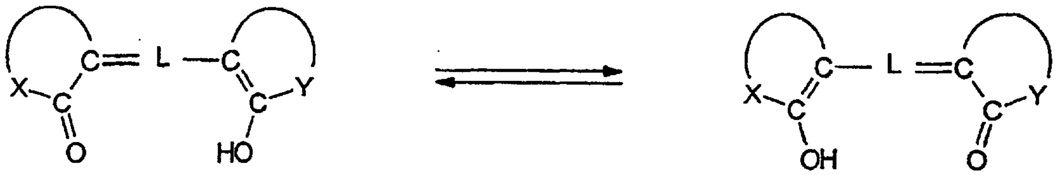

(Ia) (Ib)

wobei in der allgemeinen Formel (Ia)/(Ib) **X** und **Y** unabhängig voneinander jeweils gemeinsam mit den beiden Kohlenstoffatomen des in der Formel (Ia)/(Ib) angegebenen Ringsystems die für die Bildung einer Pyrazolongruppe, Pyridongruppe, Dioxothiazolingruppe, Rhodaningruppe, Dioxoimidazolidingruppe oder Barbitursäuregruppe notwendigen Elemente darstellen und L für eine Brückengruppe der allgemeinen Formel

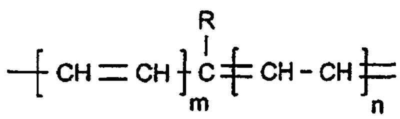

steht, worin **R** ein Wasserstoffatom, eine Phenylgruppe, eine Methylgruppe, eine Carbonamidgruppe oder ein Halogenatom darstellt und die Indices **m** und **n** jeweils gleich 0, 1 oder 2 sein können, wobei die Summe aus m und n nicht größer als 2 ist.

**2.** Mittel nach Anspruch 1, **dadurch gekennzeichnet, daß L** eine Monomethineinheit, Trimethineinheit oder Pentamethineinheit darstellt.

**3.** Mittel nach Anspruch 1 oder 2, **dadurch gekennzeichnet, daß** die Oxonolfarbstoffe der Formel (Ia)/(Ib) ausgewählt sind aus Oxonolfarbstoffen der allgemeinen Formeln (II) bis (IV),

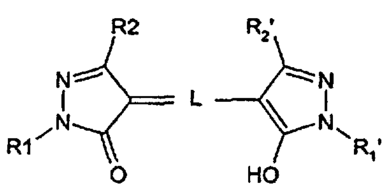

(II)

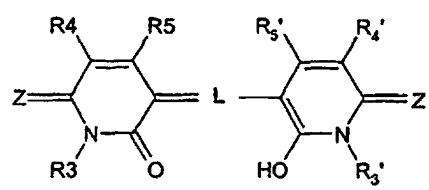

(III)

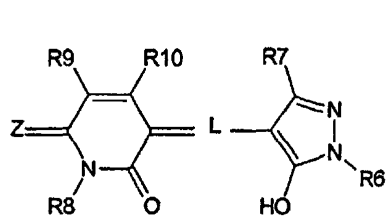

(IV)

worin **R1, R1' und R6** Wasserstoff, eine geradkettige oder verzweigte C1- bis C8- Alkylgruppe, eine Hydroxyethylgruppe, eine Dihydroxypropylgruppe, eine Methoxyethylgruppe, eine Carboxyethylgruppe, eine C1- bis C4-Sulfoalkylgruppe, einen Phenylrest oder einen mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierten Phenylrest, einen Benzylrest oder einen mit einem oder mehreren Halogenatomen, mit einer C1- bis C4-Alkylgruppe, einer Hydroxygruppe, einer Methoxygruppe, einer Carboxygruppe, einer Nitrogruppe oder einer Aminogruppe substituierten Benzylrest oder einen fünfgliedrigen oder sechsgliedrigen gesättigten oder ungesättigten Heterocyclus bedeuten, wobei **R1 und R1'** gleich oder verschieden sein können, und

**R2, R2' und R7** Wasserstoff, eine verzweigte oder unverzweigte C1- bis C6- Alkylgruppe, einen Phenylrest, eine Aminogruppe, die zusätzlich acyliert oder sulfonyliert sein kann, eine Acetylgruppe, eine Methoxygruppe, eine Carbonsäuregruppe, die mit einem geradkettigen oder verzweigten C1- bis C8-Alkohol oder einem Ethylenglykolmonomethylether oder einem Ethylenglykolmonoethylether verestert sein kann, eine Carbonsäureamidgruppe, eine Carbonsäureanilidgruppe, eine 2-Amino-2-oxyethylgruppe oder eine Nitrilgruppe bedeuten, wobei **R2 und R2'** gleich oder verschieden sein können, und

**R3, R3' und R8** Wasserstoff, eine geradkettige oder verzweigte C1- bis C11-Alkylgruppe, eine geradkettige oder verzweigte C1- bis C11-Monohydroxyalkylgruppe, eine geradkettige oder verzweigte C1- bis C11-Dihydroxyalkylgruppe, eine geradkettige oder verzweigte C1- bis C11-Alkoxyalkylgruppe, eine geradkettige oder verzweigte C1- bis C11-Monoalkylaminogruppe, eine Aminogruppe der Formel $(CH_2)_x$-NR11R12 (mit x gleich einer ganzen Zahl von 0 bis 3 und R11 und R12 unabhängig voneinander gleich einer C1- bis C3-Alkylgruppe), eine C2- bis C4-Sulfoalkylgruppe oder Carboxyalkylgruppe, eine Phenylgruppe oder eine mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierte Phenylgruppe, eine Benzylgruppe oder eine mit einem oder mehreren Halogenatomen, mit einer C1- bis C4-Alkylgruppe, einer Hydroxygruppe, einer Methoxygruppe, einer Nitrogruppe oder einer Aminogruppe substituierte Benzylgruppe, eine Phenylethylgruppe, ein fünfgliedriger oder sechsgliedriger aromatischer oder nicht-aromatischer Heterocyclus, der direkt oder über eine Methylengruppe gebunden sein kann, eine Pyrrolidinogruppe, Morpholinogruppe, Piperazinogruppe, Piperidinogruppe oder Pyridino(C2- oder C3-)alkylgruppe oder eine Trialkylammoniumalkylgruppe der Formel $R13\text{-}N(R14)_3^+$ (mit R13 gleich einer C1- bis C6-Alkylengruppe und R14 gleich einer Methyl- oder Ethylgruppe, wobei die Gesamtzahl an Kohlenstoffatomen im Molekül gleich 5 bis 9 ist) bedeuten, wobei **R3 und R3'** gleich oder verschieden sein können, und

**R4, R4' und R9** Wasserstoff, eine Nitrilgruppe, eine Carbonsäureestergruppe, eine Carbonsäureamidgruppe, eine Sulfonsäuregruppe, eine Sulfomethylgruppe, eine Methansulfonylgruppe, einen Pyridiniumrest oder einen Imidazoliumrest bedeuten, wobei **R4 und R4'** gleich oder verschieden sein können, und

**R5, R5' und R10** Wasserstoff, eine C1- bis C4-Alkylgruppe, eine C5- bis C6-Cycloalkylgruppe, eine Phenylgruppe, eine Methoxyphenylgruppe, eine Benzylgruppe, eine Phenylethylgruppe oder eine Carbonsäuregruppe bedeuten, wobei **R5 und R5'** gleich oder verschieden sein können, und

**Z** für Sauerstoff oder einen Rest der Formel $C(CN)_2$, C(CN)COOQ oder $C(COOQ)_2$, worin Q jeweils eine C1- bis C8-Alkylgruppe oder einen Ethylenglykolmono(C3- bis C7-)alkylether darstellt, steht, und

**L** für eine Brückengruppe der allgemeinen Formel

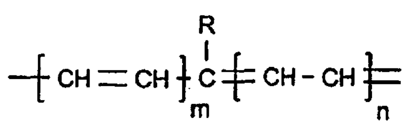

steht, worin **R** ein Wasserstoffatom, eine Phenylgruppe, eine Methylgruppe, eine Carbonamidgruppe oder ein Halogenatom darstellt, und die Indices **m** und **n** jeweils gleich 0, 1 oder 2 sein können, wobei die Summe aus m und n nicht größer als 2 ist.

**4.** Mittel nach Anspruch 3, **dadurch gekennzeichnet, daß** die Oxonolfarbstoffe der Formel (II) bis (IV) ausgewählt sind aus 4-(5-Hydroxy-1,3-dimethyl-1H-pyrazol-4-ylmethylen)-2,5-dimethyl-2,4-dihydropyrazol-3-on; 2-(2-Hydroxyethyl)-4-(5-hydroxy-1-(2-hydroxyethyl)-3-methyl-1H-pyrazol-4-ylmethylen)-5-methyl-2,4-dihydropyrazol-3-on; 4-(5-Hydroxy-3-methyl-1-(4-sulfophenyl)-1H-pyrazol-4-ylmethylen)-5-methyl-2-(4-sulfophenyl)-2,4-dihydropyrazol-3-on; Diammonium-4-(5-hydroxy-3-methyl-1-(4-sulfophenyl)-1H-pyrazol-4-yl-methylen)-5-methyl-2-(4-sulfophenyl)-2,4-dihydro-pyrazol-3-on; 4-(3-(5-Hydroxy-1,3-dimethyl-1H-pyrazol-4-yl)-allyliden)-2,5-dimethyl-2,4-dihydropyrazol-3-on; 5-Amino-4-(3-(3-amino-5-hydroxy-1-phenyl-1H-pyrazol-4-yl)-allyliden)-2-phenyl-2,4-dihydropyrazol-3-on; 2-(2-Hydroxyethyl)-4-(3-(5-hydroxy-1-(2-hydroxyethyl)-3-methyl-1H-pyrazol-4-yl)-allyliden)-5-methyl-2,4-dihydropyrazol-3-on; 4-(3-(5-Hydroxy-3-methyl-1-(4-sulfophenyl)-1H-pyrazol-4-yl)-allyliden)-5-methyl-2-(4-sulfophenyl)-2,4-dihydro-pyrazol-3-on; Dinatrium-4-(3-(5-hydroxy-3-methyl-1-(4-sulfophenyl)-1H-pyrazol-4-yl)-allyliden)-5-methyl-2-(4-sulfophenyl)-2,4-dihydro-pyrazol-3-on; 4-(3-(5-Hydroxy-3-methyl-1-(4-sulfophenyl)-1H-pyrazol-4-yl)-but-2-enyliden)-5-methyl-2-(4-sulfophenyl)-2,4-dihydropyrazol-3-on; Trikalium-4-(3-(5-hydroxy-3-methyl-1-(4-sulfophenyl)-1H-pyrazol-4-yl)-but-2-enyliden)-5-methyl-2-(4-sulfophenyl)-2,4-dihydro-pyrazol-3-on; 4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure; Tetranatrium-4-(3-(3-Carboxy-5-oxo-1 -(4-sulfophenyl)-1,5-dihydropyrazol-4-yliden)-propenyl)-5-hydroxy-1-(4-sulfophenyl)-1H-pyrazol-3-carbonsäure; 5-Hydroxy-4-(3-(3-methoxycarbonyl-5-oxo-1 -(4-sulfophenyl)-1,5-dihydropyrazol-4-yliden)-propenyl)-1-(4-sulfophenyl)-1H-pyrazol-3-carbonsäuremethylester; 4-(5-(5-Hydroxy-1,3-dimethyl-1H-pyrazol-4-yl)-penta-2,4-dienyliden)-2,5-dimethyl-2,4-dihydropyrazol-3-on; 5-Amino-4-(5-(3-amino-5-hydroxy-1-phenyl-1Hpyrazol-4-yl)-penta-2,4-dienyliden)-2-phenyl-2,4-dihydropyrazol-3-on; 2-(2-Hydroxyethyl)-4-(5-(5-hydroxy-1-(2-hydroxyethyl)-3-methyl-1Hpyrazol-4-yl)-penta-2,4-dienyliden)-5-methyl-2,4-dihydro-pyrazol-3-on; 4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on; Dikalium-4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on; 4-(5-(3-Carboxy-5-oxo-1-(4-sulfophenyl)-1,5-dihydropyrazol-4-yliden)-penta-1,3-dienyl)-5-hydroxy-1-(4-sulfophenyl)-1H-pyrazol-3-carbonsäure; Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure; 6-Hydroxy-1-(2-hydroxyethyl)-5-(3-(1-(2-hydroxyethyl)-3-methyl-5-oxo-1,5-dihydro-pyrazol-4-yliden)-propenyl)-4-methyl-2-oxo-1,2-dihydro-pyridin-3-carbonitril; 6-Hydroxy-5-(3-(1-(2-hydroxyethyl)-3-methyl-5-oxo-1,5-dihydropyrazol-4-yliden)-propenyl)-4-methyl-2-oxo-1-(4-sulfophenyl)-1,2-dihydropyridin-3-carbonitril; Kalium-6-hydroxy-5-(3-(1-(2-hydroxyethyl)-3-methyl-5-oxo-1,5-dihydro-pyrazol-4-yliden)-propenyl)-4-methyl-2-oxo-1-(4-sulfophenyl)-1,2-dihydro-pyridin-3-carbonitril; 6-Hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril; Dikalium-6-hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril; 6-Hydroxy-4-methyl-5-(3-(3-methyl-5-oxo-1-phenyl-1,5-dihydropyrazol-4-yliden)-propenyl)-2-oxo-1-(3-sulfophenyl)-1,2-dihydropyridin-3-carbonitril; 4-(3-(5-Cyano-2-hydroxy-4-methyl-6-oxo-1-phenyl-1,6-dihydropyridin-3-yl)-allyliden)-5-oxo-1-(4-sulfophenyl)-4,5-dihydro-1H-pyrazol-3-carbonsäure; 6-Hydroxy-5-(3-(1-(2-hydroxyethyl)-3-methyl-5-oxo-1,5-dihydropyrazol-4-yliden)-propenyl)-4-methyl-2-oxo-1-phenyl-1,2-dihydropyridin-3-carbonitril; 5-(3-(4-Carboxy-2,6-dioxo-1,6-dihydro-2H-pyridin-3-yliden)-propenyl)-6-hydroxy-2-oxo-1,2-dihydropyridin-4-carbonsäure: 2-(3-Cyano-5-(3-(5-cyano-6-dicyanomethylen-4-methyl-2-oxo-1,6-dihydro-2H-pyridin-3-yliden)-propenyl)-6-hydroxy-4-methyl-1H-pyridin-2-yliden)-malononitril; 5-(3-(5-Cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril; Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril; 5-(3-(5-Cyano-1-(2-hydroxyethyl)-4-methyl-2,6-dioxo-1,6-dihydro-2H-pyridin-3-yliden)-propenyl)-6-hydroxy-1-(2-hydroxyethyl)-4-methyl-2-oxo-1,2-dihydropyridin-3-carbonitril; 5-(3-(5-Cyano-1-ethyl-4-(4-methoxyphenyl)-2,6-dioxo-1,6-dihydro-2Hpyridin-3-yliden)-propenyl)-1-ethyl-6-hydroxy-4-(4-methoxyphenyl)-2-oxo-1,2-dihydropyridin-3-carbonitril; 1-Butyl-5-(3-(1-butyl-5-cyano-4-methyl-2,6-dioxo-1,6-dihydro-2H-pyridin-3-yliden)-propenyl)-6-hydroxy-4-methyl-2-oxo-1,2-dihydropyridin-3-carbonitril; 5-(3-(5-Cyano-1-(3-methoxypropyl)-4-methyl-2,6-dioxo-1,6-dihydro-2H-pyridin-3-yliden)-propenyl)-6-hydroxy-1-(3-methoxypropyl)-4-methyl-2-oxo-1,2-dihydropyridin-3-carbonitril; 5-(3-(5-Cyano-1-cyclohexyl-4-methyl-2,6-dioxo-1,6-dihydro-2Hpyridin-3-yliden)-propenyl)-1-ethyl-6-hydroxy-4-methyl-2-oxo-1,2-dihydropyridin-3-carbonitril; 5-(3-(5-Cyano-4-methyl-1-(2-morpholin-4-ylethyl)-2,6-dioxo-1,6-dihydro-2H-pyridin-3-yliden)-propenyl)-6-hydroxy-4-methyl-1-(2-morpholin-4-yl-ethyl)-2-oxo-1,2-dihydropyridin-3-carbonitril; 5-(3-(5-Cyano-4-methyl-2,6-dioxo-1-(4-sulfophenyl)-1,6-dihydro-2Hpyridin-3-yliden)-propenyl)-6-hydroxy-4-methyl-2-oxo-1-(4-sulfophenyl)-1,2-dihydropyridin-3-carbonitril; 5-(3-(5-Cyano-4-methyl-2,6-dioxo-1-(3-sulfophenyl)-1,6-dihydro-2H-pyridin-3-yliden)-propenyl)-6-hydroxy-4-methyl-2-oxo-1-(3-sulfophenyl)-1,2-dihydropyridin-3-carbonitril; 5-(3-(5-Cyano-1-dimethylamino-4-methyl-2,6-dioxo-1,6-dihydro-2Hpyridin-3-yliden)-propenyl)-1-dimethylamino-6-hydroxy-4-methyl-2-oxo-1,2-dihydropyridin-3-carbonitril; 5-(3-(5-Carbamoyl-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonsäureamid; 1-Ethyl-3-(3-(1-ethyl-2-hydroxy-5-methansulfonyl-4-methyl-6-oxo-1,6-dihydro-pyridin-3-yl)-allyliden)-5-methansulfonyl-4-methyl-3H-pyridin-2,6-dion und 5-(5-(5-Cyano-1-ethyl-4-methyl-2,6-dioxo-1,6-dihydro-2H-pyridin-3-yliden)-penta-1,3-dienyl)-1-ethyl-6-hydroxy-4-methyl-2-oxo-1,2-dihydropyridin-3-carbonitril.

5. Mittel nach einem der Ansprüche 1 bis 4, **dadurch gekennzeichnet, daß** das physiologisch verträgliche Salz ausgewählt ist aus Ammoniumsalzen, Natriumsalzen, Kaliumsalzen, N-Methylmorpholiniumsalzen, Monoethanolammoniumsalzen, Diethanolammoniumsalzen und Triethanolammoniumsalzen.

6. Mittel nach einem der Ansprüche 1 bis 5, **dadurch gekennzeichnet, daß** es 0,01 bis 5 Gewichtsprozent der Oxonolfarbstoffe der Formeln (Ia/Ib) bis (IV) enthält.

**7.** Mittel nach einem der Ansprüche 1 bis 6, **dadurch gekennzeichnet, daß** es einen pH-Wert von 2 bis 11 aufweist.

**8.** Mittel nach einem der Ansprüche 1 bis 7, **dadurch gekennzeichnet, daß** es zusätzlich natürliche oder synthetische Polymere oder modifizierte Polymere natürlichen Ursprungs enthält.

**9.** Verfahren zum temporären Färben von Haaren, **dadurch gekennzeichnet, daß** 50 bis 150 Gramm eines Mittels nach einem der Ansprüche 1 bis 8 auf das Haar aufgetragen werden und nach einer Einwirkungszeit von 10 bis 45 Minuten bei 20 bis 50 °C das Haar mit Wasser gespült wird, gegebenenfalls mit einem Shampoo gewaschen und/oder mit der wäßrigen Lösung einer schwachen organischen Säure nachgespült und sodann getrocknet wird, und das gefärbte Haar zu einem späteren Zeitpunkt mit Hilfe eines Reduktionsmittels oder Oxidationsmittels wieder entfärbt wird.

**10.** Verfahren zum temporären Färben von Haaren, **dadurch gekennzeichnet, daß** das Haar mit einem Mittel nach Anspruch 8 befeuchtet wird, zur Frisur gelegt und anschließend getrocknet wird, und das gefärbte Haar zu einem späteren Zeitpunkt mit Hilfe eines Reduktionsmittels oder Oxidationsmittels wieder entfärbt wird.

**11.** Verfahren nach Anspruch 9 oder 10, **dadurch gekennzeichnet, daß** als Reduktionsmittel Alkalisulfite, Alkalihydrogensulfite, Alkalipyrosulfite, Ammoniumsulfit oder Ammoniumhydrogensulfit, verwendet werden.

**12.** Verfahren nach Anspruch 9 oder 10, **dadurch gekennzeichnet, daß** als Reduktionsmittel eine Kombination aus a) Alkalisulfiten, Alkalihydrogensulfiten, Alkalipyrosulfiten, Ammoniumsulfit oder Ammoniumhydrogensulfit und b) Reduktonen und/oder Thiolen, verwendet wird.

**13.** Verfahren nach Anspruch 9 oder 10, **dadurch gekennzeichnet, daß** als Oxidationsmittel 5 bis 50 Gewichtsprozent Ammoniumpersulfat und/oder Alkalipersulfate enthaltende Blondierpulver, alleine oder in Kombination mit einer Wasserstoffperoxidlösung, verwendet werden.

**14.** 2-Komponenten-Kit zum Färben und späteren Entfärben der Haare, bestehend aus (a) einem Haarfärbemittel und (b) einem Entfärbemittel, **dadurch gekennzeichnet, daß** als Haarfärbemittel (a) ein Mittel nach einem der Ansprüche 1 bis 8 und als Entfärbemittel (b) ein Reduktionsmittel oder Oxidationsmittel verwendet wird.

## Claims

**1.** Agent for colouring keratin fibres, **characterized in that** it comprises at least one oxonol dye of the tautomeric formula (Ia)/(Ib) or physiologically compatible salts thereof,

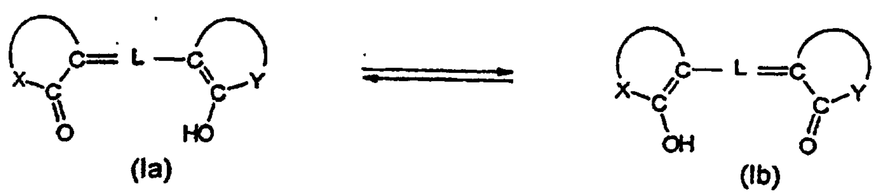

(Ia) (Ib)

where, in the general formula (Ia)/(Ib), **X** and **Y,** independently of one another, in each case together with the two carbon atoms of the ring system given in formula (Ia)/(Ib), represent the elements necessary for the formation of a pyrazolone group, pyridone group, dioxothiazoline group, rhodanine group, dioxoimidazolidine group or barbituric acid group, and **L** is a bridging group of the general formula

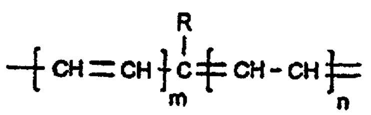

in which **R** is a hydrogen atom, a phenyl group, a methyl group, a carboxamide group or a halogen atom, and the indices **m** and **n** may each be 0, 1 or 2, where the sum of m and n is not greater than 2.

2. Agent according to Claim 1, **characterized in that** L is a monomethine unit, trimethine unit or pentamethine unit.

3. Agent according to Claim 1 or 2, **characterized in that** the oxonol dyes of the formula (Ia)/(Ib) are chosen from oxonol dyes of the general formulae (II) to (IV)

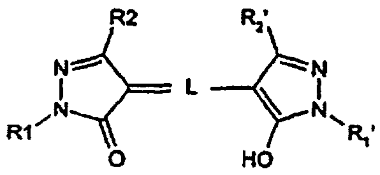

(II)

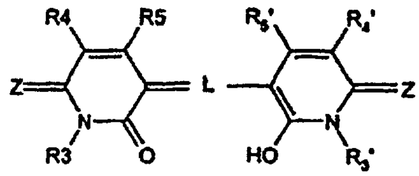

(III)

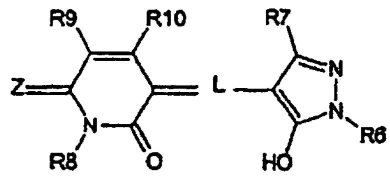

(IV)

in which **R1, R1' and R6** are hydrogen, a straight-chain or branched C1- to C8-alkyl group, a hydroxyethyl group, a dihydroxypropyl group, a methoxyethyl group, a carboxyethyl group, a C1- to C4-sulphoalkyl group, a phenyl radical or a phenyl radical substituted by one or more halogen atoms, one or two sulphonic acid groups, one or two carboxylic acid groups, one or more unbranched or branched C1- to C8-alkyl groups or C1- to C8-alkoxy groups, a benzyl radical or a benzyl radical substituted by one or more halogen atoms, by a C1- to C4-alkyl group, a hydroxyl group, a methoxy group, a carboxyl group, a nitro group or an amino group, or a five-membered or six-membered saturated or unsaturated heterocycle, where **R1 and R1'** may be identical or different, and
**R2, R2' and R7** are hydrogen, a branched or unbranched C1- to C6-alkyl group, a phenyl radical, an amino group, which may additionally be acylated or sulphonylated, an acetyl group, a methoxy group, a carboxylic acid group, which may be esterified with a straight-chain or branched C1- to C8-alcohol or an ethylene glycol monomethyl ether or an ethylene glycol monoethyl ether, a carboxamide group, a carboxanilide group, a 2-amino-2-oxyethyl group or a nitrile group, where **R2 and R2'** may be identical or different, and
**R3, R3' and R8** are hydrogen, a straight-chain or branched C1- to C11-alkyl group, a straight-chain or branched C1- to C11-monohydroxyalkyl group, a straight-chain or branched C1- to C11-dihydroxyalkyl group, a straight-chain or branched C1- to C11-alkoxyalkyl group, a straight-chain or branched C1- to C11-monoalkylamino group, an amino group of the formula $(CH_2)_x$-NR11R12 (where x is an integer from 0 to 3 and R11 and R12, independently of one another, are a C1- to C3-alkyl group), a C2- to C4-sulphoalkyl group or carboxyalkyl group, a phenyl group or a phenyl group substituted by one or more halogen atoms, one or two sulphonic acid groups, one or two carboxylic acid groups, one or more unbranched or branched C1- to C8-alkyl groups or C1- to C8-alkoxy groups, a benzyl group or a benzyl group substituted by one or more halogen atoms, by a C1- to C4-alkyl group, a hydroxyl group, a methoxy group, a nitro group or an amino group, a phenylethyl group, a five-membered or six-membered aromatic or nonaromatic heterocycle, which may be bonded directly or via a methylene group, a pyrrolidino group, morpholino group, piperazino group, piperidino group or pyridino(C2- or C3-)alkyl group or a trialkylammoniumalkyl group of the formula R13-$N(R14)_3^+$ (where R13 is a C1- to C6-alkylene group and R14 is a methyl or ethyl group, where the total number of carbon atoms in the molecule is 5 to 9), where **R3 and R3'** may be identical or different, and
**R4, R4' and R9** are hydrogen, a nitrile group, a carboxylate group, a carboxamide group, a sulphonic acid group, a sulphomethyl group, a methanesulphonyl group, a pyridinium radical or an imidazolium radical, where **R4 and R4'** may be identical or different, and
**R5, R5' and R10** are hydrogen, a C1- to C4-alkyl group, a C5- to C6-cycloalkyl group, a phenyl group, a methoxyphenyl group, a benzyl group, a phenylethyl group or a carboxylic acid group, where **R5 and R5'** may be identical or different, and

**Z** is oxygen or a radical of the formula $C(CN)_2$, C(CN)COOQ or $C(COOQ)_2$, in which Q is in each case a C1- to C8-alkyl group or an ethylene glycol mono(C3- to C7-)alkyl ether, and L is a bridging group of the general formula

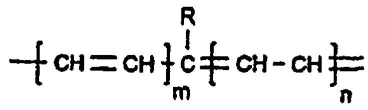

in which **R** is a hydrogen atom, a phenyl group, a methyl group, a carboxamide group or a halogen atom, and the indices **m** and **n** may each be 0, 1 or 2, where the sum of m and n is not greater than 2.

**4.** Agent according to Claim 3, **characterized in that** the oxonol dyes of the formula (II) to (IV) are chosen from 4-(5-hydroxy-1,3-dimethyl-1H-pyrazol-4-ylmethylene)-2,5-dimethyl-2,4-dihydropyrazol-3-one; 2- (2-hydroxyethyl) -4- (5-hydroxy-1-(2-hydroxyethyl)-3-methyl-1H-pyrazol-4-ylmethylene)-5-methyl-2,4-dihydropyrazol-3-one; 4-(5-hydroxy-3-methyl-1-(4-sulphophenyl)-1H-pyrazol-4-ylmethylene)-5-methyl-2-(4-sulphophenyl)-2,4-dihydropyrazol-3-one; diammonium 4-(5-hydroxy-3-methyl-1-(4-sulphophenyl)-1H-pyrazol-4-ylmethylene)-5-methyl-2-(4-sulphophenyl)-2,4-dihydropyrazol-3-one; 4-(3-(5-hydroxy-1,3-dimethyl-1H-pyrazol-4-yl)allylidene)-2,5-dimethyl-2,4-dihydropyrazol-3-one; 5-amino-4-(3-(3-amino-5-hydroxy-1-phenyl-1H-pyrazol-4-yl)allylidene)-2-phenyl-2,4-dihydropyrazol-3-one; 2-(2-hydroxyethyl)-4-(3-(5-hydroxy-1-(2-hydroxyethyl)-3-methyl-1H-pyrazol-4-yl)allylidene)-5-methyl-2,4-dihydropyrazol-3-one; 4-(3-(5-hydroxy-3-methyl-1-(4-sulphophenyl)-1H-pyrazol-4-yl)allylidene)-5-methyl-2-(4-sulphophenyl)-2,4-dihydropyrazol-3-one; disodium 4-(3-(5-hydroxy-3-methyl-1-(4-sulphophenyl)-1H-pyrazol-4-yl)allylidene)-5-methyl-2-(4-sulphophenyl)-2,4-dihydropyrazol-3-one; 4-(3-(5-hydroxy-3-methyl-1-(4-sulphophenyl)-1H-pyrazol-4-yl)but-2-enylidene)-5-methyl-2-(4-sulphophenyl)-2,4-dihydropyrazol-3-one; tripotassium 4-(3-(5-hydroxy-3-methyl-1-(4-sulphophenyl)-1H-pyrazol-4-yl)but-2-enylidene)-5-methyl-2-(4-sulphophenyl)-2,4-dihydropyrazol-3-one; 4-(3-(3-carboxy-5-oxo-1-(4-sulphophenyl)-1,5-dihydropyrazol-4-ylidene)propenyl)-5-hydroxy-1-(4-sulphophenyl)-1H-pyrazole-3-carboxylic acid, tetrasodium 4-(3-(3-carboxy-5-oxo-1-(4-sulphophenyl)-1,5-dihydropyrazol-4-ylidene)propenyl)-5-hydroxy-1-(4-sulphophenyl)-1H-pyrazole-3-carboxylic acid; methyl 5-hydroxy-4-(3-(3-methoxycarbonyl-5-oxo-1-(4-sulphophenyl)-1,5-dihydropyrazol-4-ylidene)propenyl)-1-(4-sulphophenyl)-1H-pyrazole-3-carboxylate; 4-(5-(5-hydroxy-1,3-dimethyl-1H-pyrazol-4-yl) penta-2,4-dienylidene)-2,5-dimethyl-2,4-dihydropyrazol-3-one; 5-amino-4-(5-(3-amino-5-hydroxy-1-phenyl-1H-pyrazol-4-yl)-penta-2,4-dienylidene)-2-phenyl-2,4-dihydropyrazol-3-one; 2-(2-hydroxyethyl)-4-(5-(5-hydroxy-1-(2-hydroxyethyl)-3-methyl-1H-pyrazol-4-yl)penta-2,4-dienylidene)-5-methyl-2,4-dihydropyrazol-3-one; 4-(5-(5-hydroxy-3-methyl-1-(4-sulphophenyl)-1H-pyrazol-4-yl)penta-2,4-dienylidene)-5-methyl-2-(4-sulphophenyl)-2,4-dihydropyrazol-3-one; dipotassium 4-(5-(5-hydroxy-3-methyl-1-(4-sulphophenyl)-1H-pyrazol-4-yl)penta-2,4-dienylidene)-5-methyl-2-(4-sulphophenyl)-2,4-dihydropyrazol-3-one; 4-(5-(3-carboxy-5-oxo-1-(4-sulphophenyl)-1,5-dihydropyrazol-4-ylidene)penta-1,3-dienyl)-5-hydroxy-1-(4-sulphophenyl)-1H-pyrazole-3-carboxylic acid; dipotassium 4-(5-(3-carboxy-5-oxo-1-(4-sulphophenyl)-1,5-dihydropyrazol-4-ylidene)penta-1,3-dienyl)-5-hydroxy-1-(4-sulphophenyl)-1H-pyrazole-3-carboxylic acid; 6-hydroxy-1-(2-hydroxyethyl)-5-(3-(1-(2-hydroxyethyl)-3-methyl-5-oxo-1,5-dihydropyrazol-4-ylidene)propenyl)-4-methyl-2-oxo-1,2-dihydropyridine-3-carbonitrile; 6-hydroxy-5-(3-(1-(2-hydroxyethyl)-3-methyl-5-oxo-1,5-dihydropyrazol-4-ylidene)propenyl)-4-methyl-2-oxo-1-(4-sulphophenyl)-1,2-dihydropyridine-3-carbonitrile; potassium 6-hydroxy-5-(3-(1-(2-hydroxyethyl)-3-methyl-5-oxo-1,5-dihydropyrazol-4-ylidene)propenyl)-4-methyl-2-oxo-1-(4-sulphophenyl)-1,2-dihydropyridine-3-carbonitrile; 6-hydroxy-4-methyl-5-(3-(3-methyl-5-oxo-1-(4-sulphophenyl)-1,5-dihydropyrazol-4-ylidene)propenyl)-2-oxo-1-(4-sulphophenyl)-1,2-dihydropyridine-3-carbonitrile; dipotassium 6-hydroxy-4-methyl-5-(3-(3-methyl-5-oxo-1-(4-sulphophenyl)-1,5-dihydropyrazol-4-ylidene)propenyl)-2-oxo-1-(4-sulphophenyl)-1,2-dihydropyridine-3-carbonitrile; 6-hydroxy-4-methyl-5-(3-(3-methyl-5-oxo-1-phenyl-1,5-dihydropyrazol-4-ylidene)propenyl)-2-oxo-1-(3-sulphophenyl)-1,2-dihydropyridine-3-carbonitrile; 4-(3-(5-cyano-2-hydroxy-4-methyl-6-oxo-1-phenyl-1,6-dihydropyridin-3-yl)allylidene)-5-oxo-1-(4-sulphophenyl)-4,5-dihydro-1H-pyrazole-3-carboxylic acid; 6-hydroxy-5-(3-(1-(2-hydroxyethyl)-3-methyl-5-oxo-1,5-dihydropyrazol-4-ylidene)propenyl)-4-methyl-2-oxo-1-phenyl-1,2-dihydropyridine-3-carbonitrile; 5-(3-(4-carboxy-2,6-dioxo-1,6-dihydro-2H-pyridin-3-ylidene) propenyl)-6-hydroxy-2-oxo-1,2-dihydropyridine-4-carboxylic acid; 2-(3-cyano-5-(3-(5-cyano-6-dicyanomethylene-4-methyl-2-oxo-1,6-dihydro-2H-pyridin-3-ylidene)propenyl)-6-hydroxy-4-methyl-1H-pyridin-2-ylidene)malononitrile; 5-(3-(5-cyano-1-ethyl-4-methyl-2,6-dioxo-1,6-dihydro-2H-pyridin-3-ylidene)propenyl)-1-ethyl-6-hydroxy-4-methyl-2-oxo-1,2-dihydropyridine-3-carbonitrile; triethylammonium-5-(3-(5-cyano-1-ethyl-4-methjyl-2,6-dioxo-1,6-dihydro-2H-pyridin-3-ylidene)propenyl)-1-ethyl-6-hydroxy-4-methyl-2-oxo-1,2-dihydropyridine-3-carbonitrile; 5-(3-(5-cyano-1-(2-hydroxyethyl)-4-methyl-2,6-dioxo-1,6-dihydro-2H-pyridin-3-ylidene)propenyl)-6-hydroxy-1-(2-hydroxyethyl)-4-methyl-2-oxo-1,2-dihydropyridine-3-carbonitrile; 5-(3-(5-cyano1-ethyl-4-(4-methoxyphenyl)-2,6-dioxo-1,6-dihydro-2H-pyridin-3-ylidene)propenyl)-1-ethyl-6-hydroxy-4-(4-methoxyphenyl)-2-oxo-1,2-dihydropyridine-3-carbonitrile; 1-butyl-5-(3-(1-butyl-5-cyano-4-methyl-2,6-dioxo-1,6-dihydro-2H-pyridin-3-ylidene)propenyl)-6-hydroxy-4-methyl-2-oxo-1,2-dihydropyridine-3-carbonitrile; 5-(3-(5-cyano-1-(3-methoxypropyl)-4-methyl-2,6-dioxo-1,6-dihydro-2H-pyridin-3-ylidene)propenyl)-6-hydroxy-1-(3-methoxypropyl)-4-methyl-2-oxo-1,2-dihydropyridine-3-carbonitrile; 5-(3-(5-cyano-1-cyclohexyl-4-methyl-2,6-dioxo-1,6-dihydro-2H-pyridin-3-ylidene)propenyl)-1-ethyl-6-hydroxy-4-methyl-2-oxo-1,2-dihydropyridine-3-carbonitrile; 5-(3-(5-cyano-4-methyl-1-(2-morpholin-4-ylethyl)-2,6-dioxo-1,6-dihydro-2H-pyridin-3-ylidene) propenyl)-6-hydroxy-4-methyl-1-(2-morpholin-4-ylethyl)-2-oxo-1,2-dihydropyridine-3-carbonitrile; 5-(3-(5-cyano-4-methyl-2,6-dioxo-1-(4-sulphophenyl)-1,6-dihydro-2H-pyridin-3-ylidene) propenyl)-6-hydroxy-4-methyl-2-oxo-1-(4-sulphophenyl)-1,2-dihydropyridine-3-carbonitrile; 5-(3-(5-cyano-4-methyl-2,6-dioxo-1-(3-sulphophenyl)-1,6-dihydro-2H-pyridin-3-ylidene)propenyl)-6-hydroxy-4-methyl-2-oxo-1-(3-sulphophenyl)-1,2-dihydropyridine-3-carbonitrile; 5-(3-(5-cyano-1-dimethylamino-4-methyl-2,6-dioxo-1,6-dihydro-2H-pyridin-3-ylidene)propenyl)-1-dimethylamino-6-hydroxy-4-methyl-2-oxo-1,2-dihydropyridine-3-carbonitrile; 5-(3-(5-carbamoyl-1-ethyl-4-methyl-2,6-dioxo-1,6-dihydro-2H-pyridin-3-ylidene)propenyl)-1-ethyl-6-hydroxy-4-methyl-2-oxo-1,2-dihydropyridine-3-carboxamide; 1-ethyl-3-(3-(1-ethyl-2-hydroxy-5-methanesulphonyl-4-methyl-6-oxo-1,6-dihydropyridin-3-yl)allylidene)-5-methanesulphonyl-4-methyl-3H-pyridin-2,6-dione and 5-(5-(5-cyano-1-ethyl-4-methyl-2,6-dioxo-1,6-dihydro-2H-pyridin-3-ylidene)penta-1,3-dienyl)-1-ethyl-6-hydroxy-4-methyl-2-oxo-1,2-dihydropyridine-3-carbonitrile.

**5.** Agent according to one of Claims 1 to 4, **characterized in that** the physiologically compatible salt is chosen from ammonium salts, sodium salts, potassium salts, N-methylmorpholinium salts, monoethanolammonium salts, diethanolammonium salts and triethanolammonium salts.

**6.** Agent according to one of Claims 1 to 5, **characterized in that** it comprises 0.01 to 5 per cent by weight of the oxonol dyes of the formulae (Ia/Ib) to (IV).

**7.** Agent according to one of Claims 1 to 6, **characterized in that** it has a pH of from 2 to 11.

**8.** Agent according to one of Claims 1 to 7, **characterized in that** it additionally comprises natural or synthetic polymers or modified polymers of natural origin.

**9.** Method of temporarily colouring hair, **characterized in that** 50 to 150 grams of an agent according to one of Claims 1 to 8 are applied to the hair and, after a contact time of from 10 to 45 minutes at 20 to 50°C, the hair is rinsed with water, optionally washed with a shampoo and/or after-rinsed with an aqueous solution of a weak organic acid and then dried, and the coloured hair is decoloured again at a later time using a reducing agent or oxidizing agent.

**10.** Method of temporarily colouring hair, **characterized in that** the hair is dampened with an agent according to Claim 8, arranged in a style and then dried, and the coloured hair is decoloured again at a later time using a reducing agent or oxidizing agent.

**11.** Method according to Claim 9 or 10, **characterized in that** the reducing agents used are alkali metal sulphites, alkali metal hydrogensulphites, alkali metal pyrosulphites, ammonium sulphite or ammonium hydrogensulphite.

**12.** Method according to Claim 9 or 10, **characterized in that** the reducing agent used is a combination of a) alkali metal sulphites, alkali metal hydrogensulphites, alkali metal pyrosulphites, ammonium sulphite or ammonium hydrogensulphite and b) reductones and/or thiols.

**13.** Method according to Claim 9 or 10, **characterized in that** the oxidizing agents used are blonding powders comprising 5 to 50 percent by weight of ammonium persulphate and/or alkali metal persulphates, alone or in combination with a hydrogen peroxide solution.

**14.** 2-Component kit for the colouring and subsequent decolouring of hair, consisting of (a) a hair colorant and (b) a decolorant, **characterized in that** the hair colorant (a) used is an agent according to one of Claims 1 to 8, and the decolorant (b) used is a reducing agent or oxidizing agent.

## Revendications

**1.** Produit pour la teinture de fibres de kératine, **caractérisé en ce qu'**il contient au moins un colorant oxonol de

formule tautomère (Ia)/(Ib) ou ses sels physiologiquement acceptables,

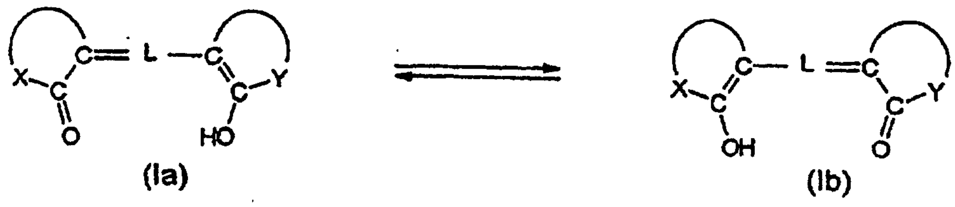

(Ia) (Ib)

où dans la formule générale (Ia)/(Ib) X et Y représentent chacun, indépendamment l'un de l'autre, conjointement avec les deux atomes de carbone du système cyclique indiqué dans la formule (Ia)/(Ib), les éléments requis pour la formation d'un groupe pyrazolone, d'un groupe pyridone, d'un groupe dioxothiazoline, d'un groupe rhodanine, d'un groupe dioxo-imidazolidine ou d'un groupe acide barbiturique, et L représente un groupe pontant de formule générale

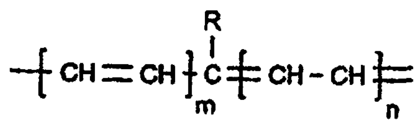

dans laquelle R représente un atome d'hydrogène, un groupe phényle, un groupe méthyle, un groupe carbamoyle ou un atome d'halogène, et les indices m et n peuvent être chacun 0, 1 ou 2, la somme de m et n n'étant pas supérieure à 2.

2. Produit selon la revendication 1, **caractérisé en ce que** L représente un groupe monométhine, triméthine ou pentaméthine.

3. Produit selon la revendication 1 ou 2, **caractérisé en ce que** les colorants oxonol de formule (Ia)/(Ib) sont choisis parmi des colorants oxonol de formules générales (II) à (IV)

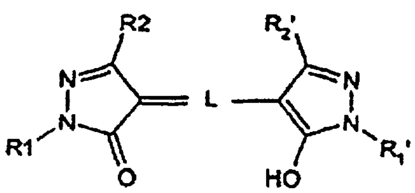

(II)

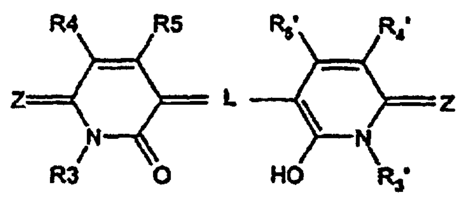

(III)

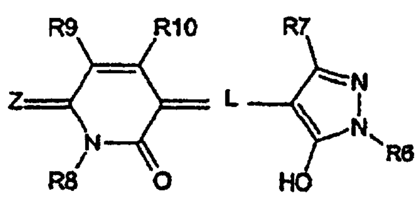

(IV)

dans lesquelles R1, R1' et R6 représentent un atome d'hydrogène, un groupe alkyle en $C_1$-$C_8$ à chaîne droite ou ramifiée, un groupe dihydroxypropyle, un groupe dihydroxypropyle, un groupe méthoxyéthyle, un groupe carboxyéthyle, un groupe sulfoalkyle en $C_1$-$C_4$, un radical phényle ou un radical phényle substitué par un ou plusieurs atomes d'halogène, un ou deux groupes sulfo, un ou deux groupes carboxy, un ou plusieurs groupes alkyle en $C_1$-$C_8$ ou groupes alcoxy en $C_1$-$C_8$ ramifiés ou non ramifiés, un radical benzyle ou un radical benzyle substitué par un ou plusieurs atomes d'halogène, par un groupe alkyle en $C_1$-$C_4$, un groupe hydroxy, un groupe méthoxy, un groupe carboxy, un groupe nitro ou un groupe amino, ou un hétérocycle à 5 ou 6 chaînons, saturé ou insaturé, R1 et R1' pouvant être identiques ou différents, et

R2, R2' et R7 représentent un atome d'hydrogène, un groupe alkyle en $C_1$-$C_6$ ramifié ou non ramifié, un radical phényle, un groupe amino qui peut être en outre acylé ou sulfonylé, un groupe acétyle, un groupe méthoxy, un groupe carboxy qui peut être estérifié par un alcool en $C_1$-$C_8$ à chaîne droite ou ramifiée ou par un éther monométhylique d'éthylèneglycol ou un éther monoéthylique d'éthylèneglycol, un groupe carbamoyle, un groupe carbanilide, un groupe 2-amino-2-oxyéthyle ou un groupe nitrile, R2 et R2' pouvant être identiques ou différents, et

R3, R3' et R8 représentent un atome d'hydrogène, un groupe alkyle en $C_1$-$C_{11}$ à chaîne droite ou ramifiée, un groupe monohydroxyalkyle en $C_1$-$C_{11}$ à chaîne droite ou ramifiée, un groupe dihydroxyalkyle en $C_1$-$C_{11}$ à chaîne droite ou ramifiée, un groupe alcoxyalkyle en $C_1$-$C_{11}$ à chaîne droite ou ramifiée, un groupe monoalkylamino en $C_1$-$C_{11}$ à chaîne droite ou ramifiée, un groupe amino de formule $(CH_2)_x$-NR11R12 (où x représente un nombre entier allant de 0 à 3 et R11 et R12 représentent, indépendamment l'un de l'autre, un groupe alkyle en $C_1$-$C_3$), un groupe sulfoalkyle ou carboxyalkyle en $C_2$-$C_4$, un groupe phényle ou un groupe phényle substitué par un ou plusieurs atomes d'halogène, un ou deux groupes sulfo, un ou deux groupes carboxy, un ou plusieurs groupes alkyle en $C_1$-$C_8$ ou groupes alcoxy en $C_1$-$C_8$ ramifiés ou non ramifiés, un groupe benzyle ou un groupe benzyle substitué par un ou plusieurs atomes d'halogène, par un groupe alkyle en $C_1$-$C_4$, un groupe hydroxy, un groupe méthoxy, un groupe nitro ou un groupe amino, un groupe phényléthyle, un hétérocycle aromatique ou non aromatique à 5 ou 6 chaînons, qui peut être lié directement ou par l'intermédiaire d'un groupe méthylène, un groupe pyrrolidino, un groupe morpholino, un groupe pipérazino, un groupe pipéridino ou un groupe pyridinoalkyle($C_2$ ou $C_3$) ou un groupe trialkylammonium-alkyle de formule R13-N$(R14)_3^+$ (où R13 représente un groupe alkylène en $C_1$-$C_6$ et R14 représente un groupe méthyle ou éthyle, le nombre total d'atomes de carbone dans la molécule allant de 5 à 9), R3 et R3' pouvant être identiques ou différents, et

R4, R4' et R9 représentent un atome d'hydrogène, un groupe nitrile, un groupe ester d'acide carboxylique, un groupe carbamoyle, un groupe sulfo, un groupe sulfométhyle, un groupe méthanesulfonyle, un radical pyridinium ou un radical imidazolium, R4 et R4' pouvant être identiques ou différents, et

R5, R5' et R10 représentent un atome d'hydrogène, un groupe alkyle en $C_1$-$C_4$, un groupe cycloalkyle en $C_5$-$C_6$, un groupe phényle, un groupe méthoxyphényle, un groupe benzyle, un groupe phényléthyle ou un groupe carboxy, R5 et R5' pouvant être identiques ou différents, et

Z représente un atome d'oxygène ou un radical de formule $C(CN)_2$, C(CN)COOQ ou $C(COOQ)_2$, où Q représente dans chaque cas un groupe alkyle en $C_1$-$C_8$ ou un monoalkyl($C_3$-$C_7$)éther d'éthylèneglycol, et

L représente un groupe pontant de formule générale

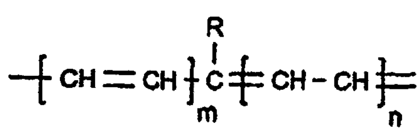

dans laquelle R représente un atome d'hydrogène, un groupe phényle, un groupe méthyle, un groupe carbamoyle ou un atome d'halogène, et les indices m et n peuvent chacun être 0, 1 ou 2, la somme de m et n n'étant pas supérieure à 2.

**4.** Produit selon la revendication 3, **caractérisé en ce que** les colorants oxonol de formules (II) à (IV) sont choisis parmi les composés suivants : 4-(5-hydroxy-1,3-diméthyl-1H-pyrazol-4-ylméthylène)-2,5-diméthyl-2,4-dihydropyrazol-3-one, 2-(2-hydroxyéthyl)-4-(5-hydroxy-1-(2-hydroxyéthyl)-3-méthyl-1H-pyrazol-4-ylméthylène)-5-méthyl-2,4-dihydropyrazol-3-one, 4-(5-hydroxy-3-méthyl-1-(4-sulfophényl)-1H-pyrazol-4-ylméthylène)-5-méthyl-2-(4-sulfophényl)-2,4-dihydropyrazol-3-one, diammonium-4-(5-hydroxy-3-méthyl-1-(4-sulfophényl)-1H-pyrazol-4-ylméthylène)-5-méthyl-2-(4-sulfophényl)-2,4-dihydropyrazol-3-one, 4-(3-(5-hydroxy-1,3-diméthyl-1H-pyrazol-4-yl)allylidène)-2,5-diméthyl-2,4-dihydropyrazol-3-one, 5-amino-4-(3-(3-amino-5-hydroxy-1-phényl-1H-pyrazol-4-yl)allylidène)-2-phényl-2,4-dihydropyrazol-3-one, 2-(2-hydroxyéthyl)-4-(3-(5-hydroxy-1-(2-hydroxyéthyl)-3-méthyl-1H-pyrazol-4-yl)allylidène)-5-méthyl-2,4-dihydropyrazol-3-one, 4-(3-(5-hydroxy-3-méthyl-1-(4-sulfophényl)-1H-pyrazol-4-yl)-allylidène)-5-méthyl-2-(4-sulfophényl)-2,4-dihydropyrazol-3-one, disodium-4-(3-(5-hydroxy-3-méthyl-1-(4-sulfophényl)-1H-pyrazol-4-yl)allylidène)-5-méthyl-2-(4-sulfophényl)-2,4-dihydropyrazol-3-one, 4-(3-(5-hydroxy-3-méthyl-1-(4-sulfophényl)-1H-pyrazol-4-yl)but-2-énylidène)-5-méthyl-2-(4-sulfophényl)-2,4-dihydropyrazol-3-one, tripotassium-4-(3-(5-hydroxy-3-méthyl-1-(4-sulfophényl)-1H-pyrazol-4-yl)but-2-énylidène)-5-méthyl-2-(4-sulfophényl)-2,4-dihydropyrazol-3-one, acide 4-(3-(3-carboxy-5-oxo-1-(4-sulfophényl)-1,5-dihydropyrazol-4-ylidène)propényl)-5-hydroxy-1-(4-sulfophényl)-1H-pyrazole-3-carboxylique, 4-(3-(3-carboxy-5-oxo-1-(4-sulfophényl)-1,5-dihydropyrazol-4-ylidène)propényl)-5-hydroxy-1-(4-sulfophényl)-1H-pyrazole-3-carboxylate tétrasodique, 5-hydroxy-4-(3-(3-méthoxycarbonyl-5-oxo-1-(4-sulfophényl)-1,5-dihydropyrazol-4-ylidène)propényl)-1-(4-sulfophényl)-1H-pyrazole-3-carboxylate de méthyle, 4-(5-(5-hydroxy-1,3-diméthyl-1H-pyrazol-4-yl)penta-2,4-diénylidène)-2,5-diméthyl-2,4-dihydropyrazol-3-one, 5-amino-4-(5-(3-amino-5-hydroxy-1-phényl-1H-pyrazol-4-yl)penta-2,4-diénylidène)-2-phényl-2,4-dihydropyrazol-3-one, 2-(2-hydroxyéthyl)-4-(5-(5-hydroxy-1-(2-hydroxyéthyl)-3-méthyl-1H-pyrazol-4-yl)penta-2,4-diénylidène)-5-méthyl-2,4-dihydropyrazol-3-one, 4-(5-(5-hydroxy-3-méthyl-1-(4-sulfophényl)-1H-pyrazol-4-yl)penta-2,4-diénylidène)-5-méthyl-2-(4-sulfophényl)-2,4-dihydropyrazol-3-one, dipotassium-4-(5-(5-hydroxy-3-méthyl-1-(4-sulfophényl)-1H-pyrazol-4-yl)penta-2,4-diénylidène)-5-méthyl-2-(4-sulfophényl)-2,4-dihydropyrazol-3-one, acide 4-(5-(3-carboxy-5-oxo-1-(4-sulfophényl)-1,5-dihydropyrazol-4-ylidène)penta-1,3-diényl)-5-hydroxy-1-(4-sulfophényl)-1H-pyrazole-3-carboxylique, 4-(5-(3-carboxy-5-oxo-1-(4-sulfophényl)-1,5-dihydropyrazol-4-ylidène)penta-1,3-diényl)-5-hydroxy-1-(4-sulfophényl)-1H-pyrazole-3-carboxylate dipotassique, 6-hydroxy-1-(2-hydroxyéthyl)-5-(3-(1-(2-hydroxyéthyl)-3-méthyl-5-oxo-1,5-dihydropyrazol-4-ylidène)propényl-4-méthyl-2-oxo-1,2-dihydropyridine-3-carbonitrile, 6-hydroxy-5-(3-(1-(2-hydroxyéthyl)-3-méthyl-5-oxo-1,5-dihydropyrazol-4-ylidène)-propényl)-4-méthyl-2-oxo-1-(4-sulfophényl)-1,2-dihydropyridine-3-carbonitrile, potassium-6-hydroxy-5-(3-(1-(2-hydroxyéthyl)-3-méthyl-5-oxo-1,5-dihydropyrazol-4-ylidène)propényl)-4-méthyl-2-oxo-1-(4-sulfophényl)-1,2-dihydropyridine-3-carbonitrile, 6-hydroxy-4-méthyl-5-(3-(3-méthyl-5-oxo-1-(4-sulfophényl)-1,5-dihydropyrazol-4-ylidène)propényl)-2-oxo-1-(4-sulfophényl)-1,2-dihydropyridine-3-carbonitrile, dipotassium-6-hydroxy-4-méthyl-5-(3-(3-méthyl-5-oxo-1-(4-sulfophényl)-1,5-dihydropyrazol-4-ylidène)propényl)-2-oxo-1-(4-sulfophényl)-1,2-dihydropyridine-3-carbonitrile, 6-hydroxy-4-méthyl-5-(3-(3-méthyl-5-oxo-1-phényl-1,5-dihydropyrazol-4-ylidène)propényl)-2-oxo-1-(3-sulfophényl)-1,2-dihydropyridine-3-carbonitrile, acide 4-(3-(5-cyano-2-hydroxy-4-méthyl-6-oxo-1-phényl-1,6-dihydropyridin-3-yl)allylidène)-5-oxo-1-(4-sulfophényl)-4,5-dihydro-1H-pyrazole-3-carboxylique, 6-hydroxy-5-(3-(1-(2-hydroxyéthyl)-3-méthyl-5-oxo-1,5-dihydropyrazol-4-ylidène)propényl)-4-méthyl-2-oxo-1-phényl-1,2-dihydropyridine-3-carbonitrile, acide 5-(3-(4-carboxy-2,6-dioxo-1,6-dihydro-2H-pyridin-3-ylidène)propényl)-6-hydroxy-2-oxo-1,2-dihydropyridine-4-carboxylique, 2-(3-cyano-5-(3-(5-cyano-6-dicyanométhylène-4-méthyl-2-oxo-1,6-dihydro-2H-pyridin-3-ylidène)propényl)-6-hydroxy-4-méthyl-1H-pyridin-2-ylidène)malononitrile, 5-(3-(5-cyano-1-éthyl-4-méthyl-2,6-dioxo-1,6-dihydro-2H-pyridin-3-ylidène)propényl)-1-éthyl-6-hydroxy-4-méthyl-2-oxo-1,2-dihydropyridine-3-carbonitrile, triéthylammonium-5-(3- (5-cyano-1-éthyl-4-méthyl-2,6-dioxo-1,6-dihydro-2H-pyridin-3-ylidène)propényl)-1-éthyl-6-hydroxy-4-méthyl-2-oxo-1,2-dihydropyridine-3-carbonitrile, 5-(3-(5-cyano-1-(2-hydroxyéthyl)-4-méthyl-2,6-dioxo-1,6-dihydro-2H-pyridin-3-ylidène)propényl)-6-hydroxy-1-(2-hydroxyéthyl)-4-méthyl-2-oxo-1,2-dihydropyridine-3-carbonitrile, 5-(3-(5-cyano-1-éthyl-4-(4-méthoxyphényl)-2,6-dioxo-1,6-dihydro-2H-pyridin-3-ylidène)-propényl)-1-éthyl-6-hydroxy-4-(4-méthoxyphényl)-2-oxo-1,2-dihydropyridine-3-carbonitrile, 1-butyl-5-(3-(1-butyl-5-cyano-4-méthyl-2,6-dioxo-1,6-dihydro-2H-pyridin-3-ylidène)propényl)-6-hydroxy-4-méthyl-2-oxo-1,2-dihydropyridine-3-carbonitrile, 5-(3-(5-cyano-1-(3-méthoxypropyl)-4-méthyl-2,6-dioxo-1,6-dihydro-2H-pyridin-3-ylidène)propényl)-6-hydroxy-1-(3-méthoxypropyl)-4-méthyl-2-oxo-1,2-dihydropyridine-3-carbonitrile, 5-(3-(5-cyano-1-cyclohexyl-4-méthyl-2,6-dioxo-1,6-dihydro-2H-pyridin-3-ylidène)propényl)-1-éthyl-6-hydroxy-4-méthyl-2-oxo-1,2-dihydropyridine-3-carbonitrile, 5-(3-(5-cyano-4-méthyl-1-(2-morpholin-4-yléthyl)-2,6-dioxo-1,6-dihydro-2H-pyridin-3-ylidène)-propényl)-6-hydroxy-4-méthyl-1-(2-morpholin-4-yléthyl)-2-oxo-1,2-dihydropyridine-3-carbonitrile, 5-(3-(5-cyano-4-méthyl-2,6-dioxo-1-(4-sulfophényl)-1,6-dihydro-2H-pyridin-3-ylidène)propényl)-6-hydroxy-4-méthyl-2-oxo-1-(4-sulfophényl)-1,2-dihydropyridine-3-carbonitrile, 5-(3-(5-cyano-4-méthyl-2,6-dioxo-1-(3-sulfophényl)-1,6-dihydro-2H-pyridin-3-ylidène)propényl)-6-hydroxy-4-méthyl-2-oxo-1-(3-sulfophényl)-1,2-dihydropyridine-3-carbonitrile, 5-(3-(5-cyano-1-diméthylamino-4-méthyl-2,6-dioxo-1,6-dihydro-2H-pyridin-3-ylidène)-propényl)-1-diméthylamino-6-hydroxy-4-méthyl-2-oxo-1,2-dihydropyridine-3-carbonitrile, 5-(3-(5-carbamoyl-1-éthyl-4-méthyl-2,6-dioxo-1,6-dihydro-2H-pyridin-3-ylidène)propényl)-1-éthyl-6-hydroxy-4-méthyl-2-oxo-1,2-dihydropyridine-3-carboxamide, 1-éthyl-3-(3-(1-éthyl-2-hydroxy-5-méthanesulfonyl-4-méthyl-6-oxo-1,6-dihydropyridin-3-yl)allylidène)-5-méthanesulfonyl-4-méthyl-3H-pyridine-2,6-dione et 5-(5-(5-cyano-1-éthyl-4-méthyl-2,6-dioxo-1,6-dihydro-2H-pyridin-3-ylidène)penta-1,3-diényl)-1-éthyl-6-hydroxy-4-méthyl-2-oxo-1,2-dihydropyridine-3-carbonitrile.

5. Produit selon l'une quelconque des revendications 1 à 4, **caractérisé en ce que** le sel physiologiquement acceptable est choisi parmi les sels d'ammonium, les sels de sodium, les sels de potassium, les sels de N-méthylmorpholinium, les sels de monoéthanolammonium, les sels de diéthanolammonium et les sels de triéthanolammonium.

6. Produit selon l'une quelconque des revendications 1 à 5, **caractérisé en ce qu'**il contient de 0,01 à 5 % en poids des colorants oxonol de formules (Ia/Ib) à (IV).

7. Produit selon l'une quelconque des revendications 1 à 6, **caractérisé en ce qu'**il présente un pH de 2 à 11.

8. Produit selon l'une quelconque des revendications 1 à 7, **caractérisé en ce qu'**il contient en outre des polymères naturels ou synthétiques ou des polymères d'origine naturelle, modifiés.

9. Procédé pour la teinture temporaire des cheveux, **caractérisé en ce qu'**on applique sur les cheveux de 50 à 150 grammes d'un produit selon l'une quelconque des revendications 1 à 8, et, après un temps d'action de 10 à 45 minutes à 20-50°C, on rince les cheveux à l'eau, éventuellement on les lave avec un shampooing et/ou on les soumet à un après-lavage avec la solution aqueuse d'un acide organique faible et ensuite on les sèche, et les cheveux teints sont de nouveau décolorés ultérieurement à l'aide d'un réducteur ou d'un oxydant.

10. Procédé pour la teinture temporaire des cheveux, **caractérisé en ce que** les cheveux sont humectés avec un produit selon la revendication 8, mis en plis et ensuite séchés, et les cheveux teints sont de nouveau décolorés ultérieurement à l'aide d'un réducteur ou d'un oxydant.

11. Procédé selon la revendication 9 ou 10, **caractérisé en ce qu'**on utilise comme réducteur des sulfites de métaux alcalins, des hydrogénosulfites de métaux alcalins, des pyrosulfites de métaux alcalins, du sulfite d'ammonium ou de l'hydrogénosulfite d'ammonium.

12. Procédé selon la revendication 9 ou 10, **caractérisé en ce qu'**on utilise comme réducteur une association de a) sulfites de métaux alcalins, hydrogénosulfites de métaux alcalins, pyrosulfites de métaux alcalins, sulfite d'ammonium ou hydrogénosulfite d'ammonium, et b) réducteurs et/ou thiols.

13. Procédé selon la revendication 9 ou 10, **caractérisé en ce qu'**on utilise comme oxydant de 5 à 50 % en poids de persulfate d'ammonium et/ou d'une poudre de teinture en blond contenant des persulfates de métaux alcalins, seuls ou en association avec une solution de peroxyde d'hydrogène.

14. Nécessaire à deux composants pour la teinture et la décoloration ultérieure des cheveux, constitué de (a) un produit de teinture pour cheveux et (b) un produit de décoloration, **caractérisé en ce qu'**on utilise comme produit de teinture pour cheveux (a) un produit selon l'une quelconque des revendications 1 à 8 et, en tant que produit de décoloration (b), un réducteur ou un oxydant.